# EUROPEAN PATENT APPLICATION

(11) **EP 4 768 046 A1**
(43) Date of publication of application: **01.07.2026**
(21) Application number: 24855770.4
(22) Date of filing: 19.08.2024
(51) Int. Cl.: A61K 47/68, A61K 47/65, A61K 31/4745, A61P 35/00

(54) **ANTIBODY-DRUG CONJUGATE, PREPARATION METHOD THEREFOR AND USE THEREOF**

(30) Priority: 21.08.2023 CN 202311058439
(71) Applicant: Hangzhou Adcoris Biopharma Co., Ltd, Hangzhou, Zhejiang 310052 (CN)
(72) Inventor: MIAO, Zhenwei, Hangzhou, Zhejiang, 310052 (CN); HUANG, Yunsheng, Hangzhou, Zhejiang, 310052 (CN); LI, Yaowu, Hangzhou, Zhejiang, 310052 (CN); WENG, Shanhui, Hangzhou, Zhejiang 310052 (CN)
(74) Representative: Elzaburu S.L.P.
(86) International application number: PCT/CN2024/113036
(87) International publication number: WO 2025/040047

(57) **Abstract**

The present invention relates to the technical field of biological medicines, and provides an antibody-drug conjugate obtained by means of conjugation of a compound shown as formula (I) with an antibody, a preparation method therefor and the use thereof. The antibody-drug conjugate has a good inhibition effect on the growth of solid tumors.

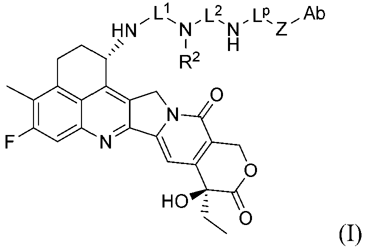

## Description

The present application claims the priority of Chinese patent application 2023110584395, filed on August 21, 2023, which is incorporated in the present application in its entirety.

### Technical Field

The present disclosure relates to the technical field of biological medicines, and in particular to an antibody-drug conjugate, a preparation method, and use thereof.

### Background Art

An antibody-drug conjugate or ADC is a novel and effective targeted drug composed of three moieties: an antibody, a linker, and a toxin. In the antibody-drug conjugate, the toxin (payload) is a key component for exerting efficacy, the antibody is a key moiety having a targeting effect, and the linker not only connects the two together, but also affects the stability of the ADC, the release mechanism of the drug, the manner of conjugating to the antibody, etc. An ideal payload can kill both targeted tumor cells and non-targeted tumor cells (without targeted antigens), i.e., a bystander killing effect, without toxicity to normal cells and tissues (without targeted antigens). An ideal linker needs to be stable in the circulatory system and release the payload upon reaching the tumor microenvironment or tumor cells (endocytosis), so as to kill the tumor cells or cells in the tumor microenvironment.

Exatecan and derivatives thereof are an effective class of DNA topoisomerase I inhibitors, which have achieved good effects as payloads in the field of the treatment of HER2-positive tumors. It is of great significance to develop more drug conjugates having anti-tumor activity in which exatecan is used as a payload.

### Summary of the Invention

The present disclosure provides a low-toxicity antibody-drug conjugate formed by linking a TOP1 isomerase inhibitor via a stable linker, which has a relatively good inhibitory effect on the growth of solid tumors.

In a first aspect, the present disclosure provides an antibody-drug conjugate represented by formula (I): 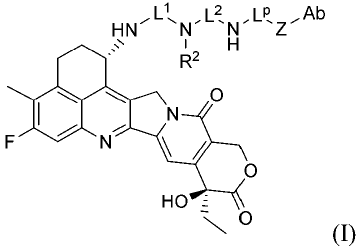 in the formula,
R² is selected from hydrogen, deuterium, C₁-C₆ alkyl, C₁-C₆ alkoxy, acyl, or sulfonyl;
L¹ is selected from -L¹¹-L¹²-L¹³-, wherein L¹¹, L¹², and L¹³ are each independently selected from absence, -C=O-, C₁-C₂ alkylene, or C₁-C₂ alkylene-O-, provided that when L¹¹ is - C=O-, R² is not hydrogen;
L² is selected from C₁-C₆ alkylene or C₁-C₆ acyl, the C₁-C₆ alkylene or C₁-C₆ acyl being optionally substituted with one or more R³;
R³ is selected from phenyl-substituted or unsubstituted C₁-C₆ alkyl and C₁-C₆ alkoxy;
L^{P} is a peptide residue composed of 1-7 amino acids, e.g., 2-7 amino acids;
Z is selected from -L^{z}-L^{j}-, wherein L^{z} is selected from absence, -C(=O)-C₁-C₈ alkylene, - C(=O)-(CH₂CH₂O)₂₋₆-CH₂CH₂NH-, or -C(=O)-(CH₂)₀₋₆-NR¹(CH₂)₀₋₆-, and L^{j} is an antibody-conjugatable linker;
R¹ is selected from -C₁-C₆ alkyl-carboxyl or -C₁-C₆ alkyl-amino; and
Ab is an antibody.

In one embodiment, the antibody is obtained by conjugating a compound of formula (I-1) (payload linker) to the antibody: 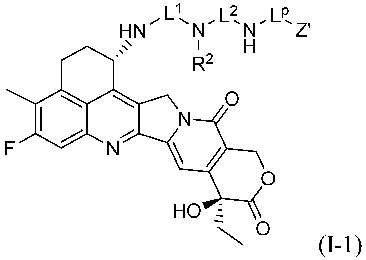 in the formula, L¹, L², R², and L^{P} are each the same as defined for the compound of formula (I).
Z' is an antibody-conjugatable linker group corresponding to Z.

In one embodiment, Z' is selected from 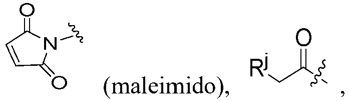 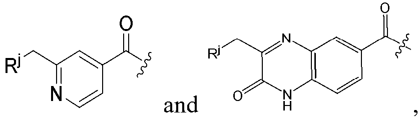 wherein R^{j} is selected from halogen, preferably bromine or iodine.

In one embodiment, Z' is selected from maleimido, bromoacetyl, or iodoacetyl.

In one embodiment, as shown in formula (I-2), the site of connection between the antibody and the payload-linker is a thiol group of a cysteine residue, for which an interchain disulfide bond of the antibody is reduced and cleaved. 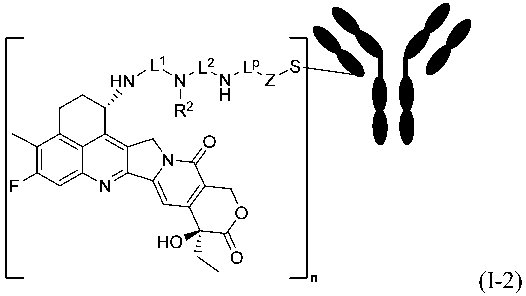 wherein 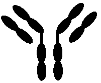 represents the antibody.

In one embodiment, the connection between the payload-linker and the cysteine of the antibody is achieved by replacing the bromine atom in the bromoacetyl linker group with the interchain thiol of the antibody, or by Michael addition for conjugation to the linker group having a maleimide structure.

In one embodiment, R² is selected from hydrogen, deuterium, C₁-C₃ alkyl, C₁-C₃ alkoxy, -C(=O)C₁-C₃ alkyl, or -S(=O)₂C₁-C₃ alkyl;
preferably, R² is selected from hydrogen, deuterium, methyl, ethyl, methoxy, ethoxy, formyl, acetyl, methylsulfonyl, or ethylsulfonyl;
preferably, R² is selected from hydrogen, methyl, methoxy, formyl, or methylsulfonyl.

In one embodiment, L¹¹, L¹², and L¹³ are each independently selected from absence, -C=O-, -CH₂-, -CH₂O-, or -OCH₂-;
preferably, L¹ is selected from -C(=O)CH₂OCH₂-, -C(=O)CH₂O-, -C(=O)CH₂, or -CH₂-;
preferably, L² is selected from C₁-C₃ alkylene;
preferably, L² is selected from methylene and ethylene.

In one embodiment, when L¹ is -C(=O)CH₂O-, R² is selected from C₁-C₆ alkyl, preferably -C₁-C₃ alkyl, more preferably methyl.

In one embodiment, when L¹ is -C(=O)CH₂-, R² is selected from C₁-C₆ alkoxy, preferably -C₁-C₃ alkoxy, more preferably methoxy.

In one embodiment, L^{p} is selected from a peptide residue composed of 1, 2, 3, or 4 amino acids selected from phenylalanine (Phe), glycine (Gly), valine (Val), alanine (Ala), or leucine (Leu);
preferably, L^{p} is selected from -Val-Cit-, -Gly-Lys-, -Gly-Leu-, -Val-Ala-, -Gly-Phe-, - GLy-Gly-Lys-, -Gly-Gly-Phe-, -Gly-Val-Ala-, -Gly-Ala-, -Gly-Gly-Val-, -Gly-Leu-Val-, -Gly-Phe-Gly-, or -Gly-Gly-Leu-;
preferably, L^{p} is selected from

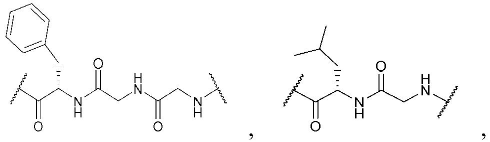

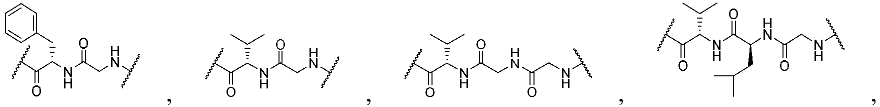

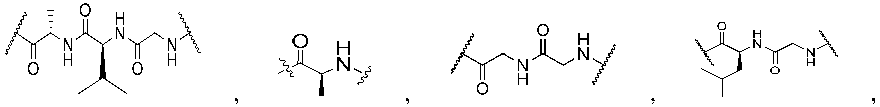

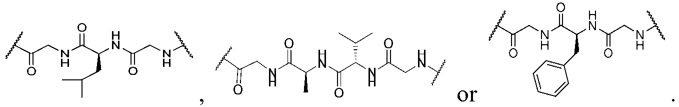

In one embodiment, L^{j} is selected from 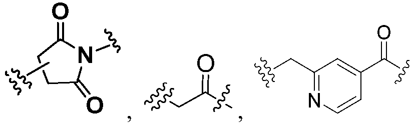 and 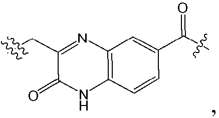 the position represented by 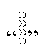 indicating where the antibody is linked, and the position represented by 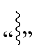 indicating where the L^{z} group is linked;
preferably, L^{z} is selected from -C(=O)-C₁-C₈ alkylene, -C(=O)-(CH₂CH₂O)₂₋₆-CH₂CH₂NH-, or -C(=O)-(CH₂)₁₋₄-NR¹(CH₂)₂-;
preferably, L^{z} is selected from -C(=O)-(CH₂CH₂O)₂₋₆-CH₂CH₂NH- or -C(=O)-(CH₂)₁₋₄-NR¹(CH₂)₂-;
preferably, R¹ is selected from -C₁-C₃ alkyl-carboxyl;
preferably, L^{z} is selected from -C(=O)-(CH₂CH₂O)₂-CH₂CH₂NH- or 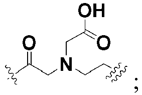
preferably, Z is selected from

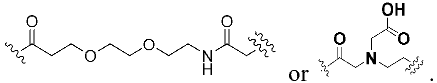

In one embodiment, the antibody-drug conjugate is obtained by conjugating a compound of formula (II) or (III) to the antibody: 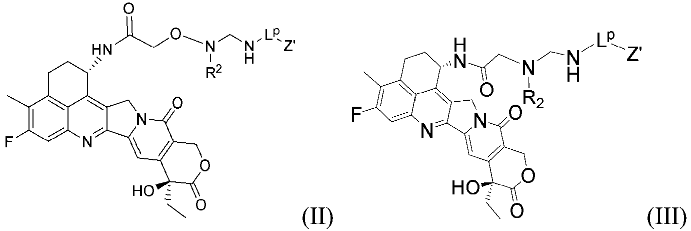
wherein R² and L^{P} are each the same as defined for the compound of formula (I), Z' is an antibody-conjugatable linker group corresponding to Z, preferably maleimido, bromoacetyl, or iodoacetyl;
preferably, the antibody-drug conjugate releases the following anti-tumor compounds in a physiological environment: 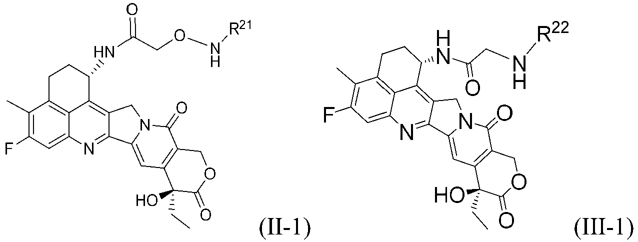
wherein R²¹ is selected from C₁-C₆ alkyl, preferably -C₁-C₃ alkyl, more preferably methyl. R²² is selected from C₁-C₆ alkoxy, preferably -C₁-C₃ alkoxy, more preferably methoxy.

In one embodiment, the physiological environment is the presence of the action of proteases.

Preferably, the following anti-tumor compounds are released:

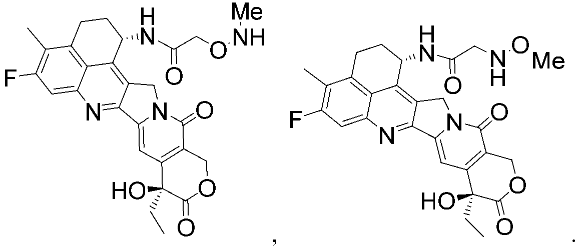

In one embodiment, the antibody is an antibody against a tumor-associated antigen; preferably, the tumor-associated antigen is selected from one or more antibodies of Her2, Nectin-4, Trop2, 5T4, B7H3, ROR1, or Claudin18.2; and the antibody may be a monospecific or multispecific antibody.

Preferably, the tumor is selected from breast cancer, lung cancer, colorectal cancer, esophageal cancer, gastric cancer, lung cancer, renal cancer, ovarian cancer, cervical cancer, bladder cancer, head and neck cancer, pancreatic cancer, or liver cancer;
preferably, the antibody is an anti-HS627 antibody, an anti-IP140B antibody, or an anti-Nectin-4 antibody.

Preferably, the antibody comprises a heavy chain having an amino acid sequence of SEQ ID NO: 1 or any variant thereof, and a light chain having an amino acid sequence of SEQ ID NO: 2 or any variant thereof;
preferably, the antibody comprises a heavy chain having an amino acid sequence of SEQ ID NO: 3 or any variant thereof, and a light chain having an amino acid sequence of SEQ ID NO: 4 or any variant thereof;
preferably, the antibody comprises a heavy chain having an amino acid sequence of SEQ ID NO: 5 or any variant thereof, and a light chain having an amino acid sequence of SEQ ID NO: 6 or any variant thereof.

In one embodiment, the drug-to-antibody ratio of the antibody-drug conjugate is 2-8, further preferably 3.5-4.5 or 7.5-8.

In one implementation, the antibody may be IgG1, IgG2, IgG3, or IgG4, preferably IgG1.

In one embodiment, the antibody-drug conjugate is obtained by conjugating the following compound to the antibody:

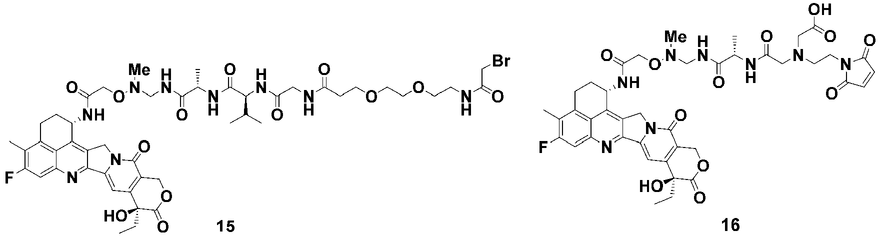

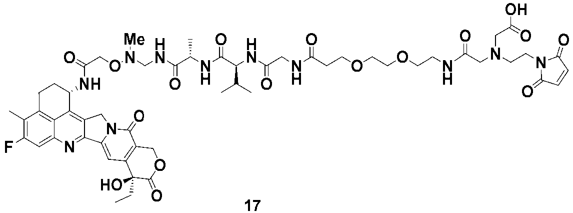

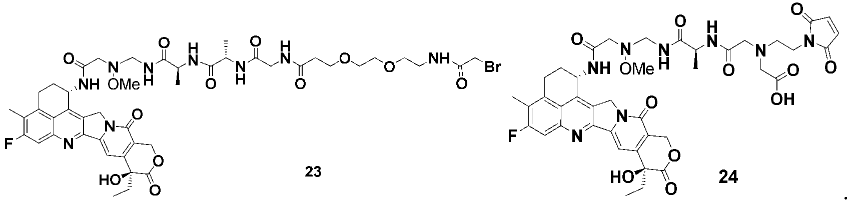

The present disclosure provides the following antibody-drug conjugates: 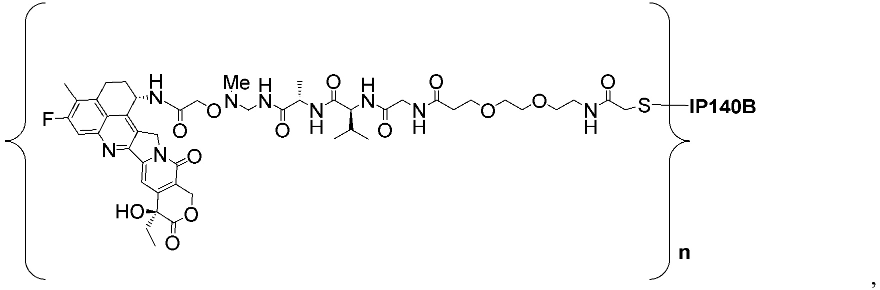 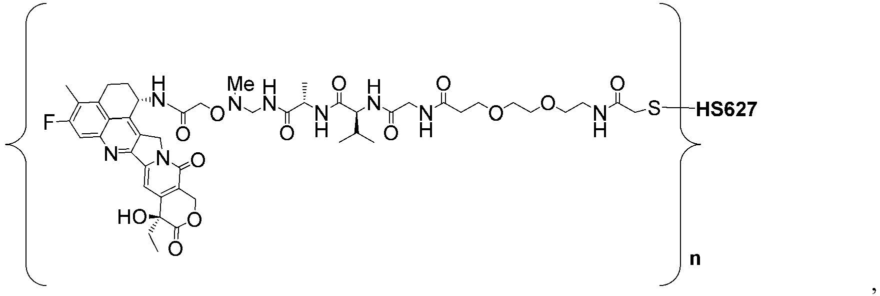 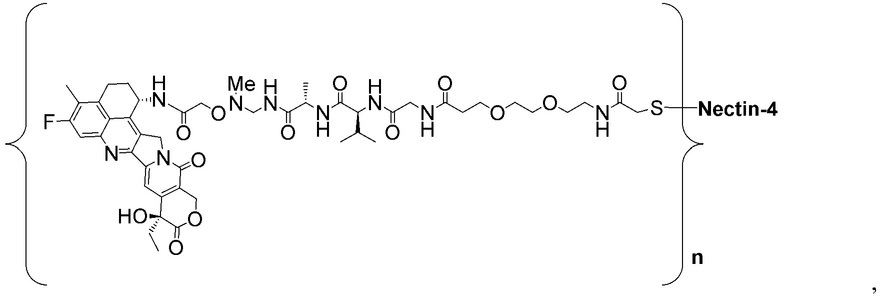 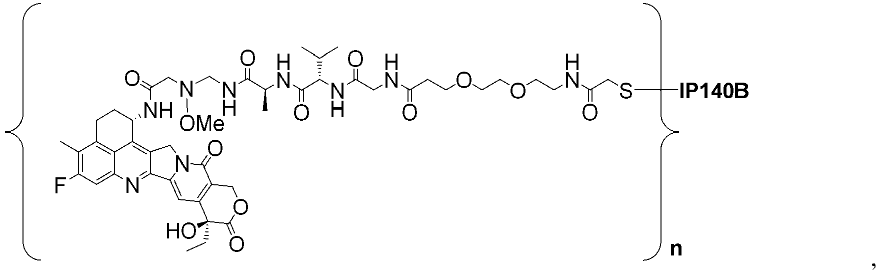 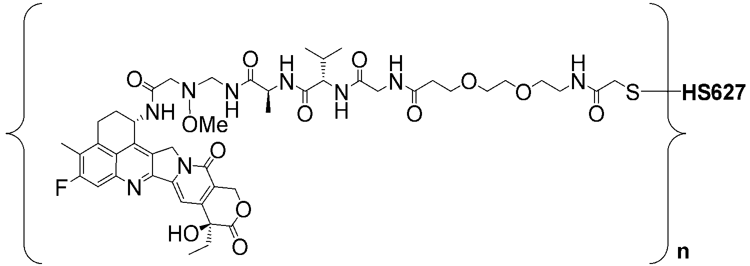 and 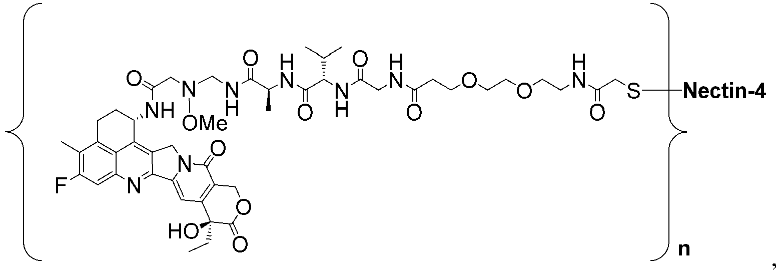 wherein n is the drug-to-antibody ratio of the antibody-drug conjugate and is preferably 8±0.5.

In another aspect, the present disclosure provides a pharmaceutical composition, comprising the antibody-drug conjugate and a pharmaceutically acceptable carrier.

In another aspect, the present disclosure provides use of the antibody-drug conjugate or the pharmaceutical composition in the manufacture of an anti-tumor drug.

In another aspect, the present disclosure provides a method for treating a tumor disease, comprising a step of administering to a patient in need thereof a therapeutically effective amount of the antibody-drug conjugate or the pharmaceutical composition.

Preferably, the tumor is a solid tumor.

Preferably, the tumor is selected from breast cancer, lung cancer, colorectal cancer, esophageal cancer, gastric cancer, lung cancer, renal cancer, ovarian cancer, cervical cancer, bladder cancer, head and neck cancer, pancreatic cancer, or liver cancer.

### Brief Description of the Drawings

FIG. 1 shows the DAR test results of ADC1 in Example 9.
FIG. 2 shows the DAR test results of ADC2 in Example 9.
FIG. 3 shows the DAR test results of ADC3 in Example 10.
FIG. 4 shows the DAR test results of ADC4 in Example 11.
FIG. 5 shows the DAR test results of ADC5 in Example 12.
FIG. 6 shows the DAR test results of ADC6 in Example 13.
FIG. 7 shows the DAR test results of ADC7 in Example 14.

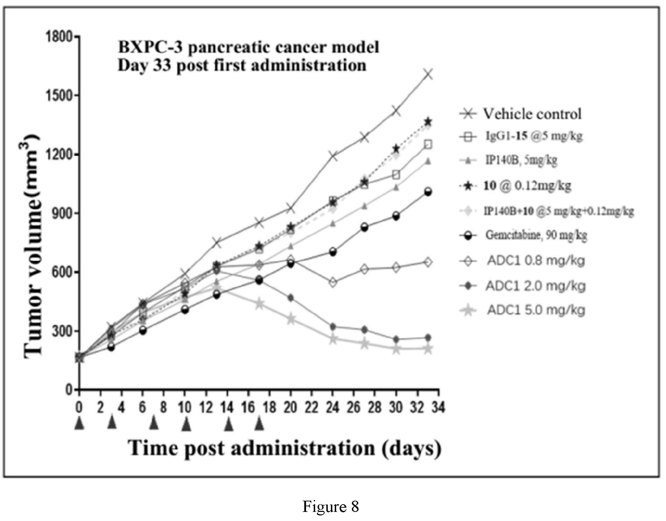

FIG. 8 shows a graph of tumor growth curves in a BXPC-3 model in Test Example 1.

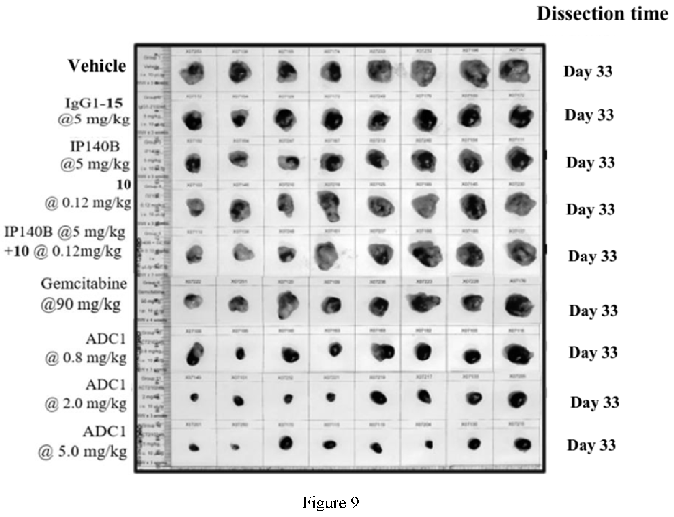

FIG. 9 shows images of tumors after dissection from the BXPC-3 model in Test Example 1.

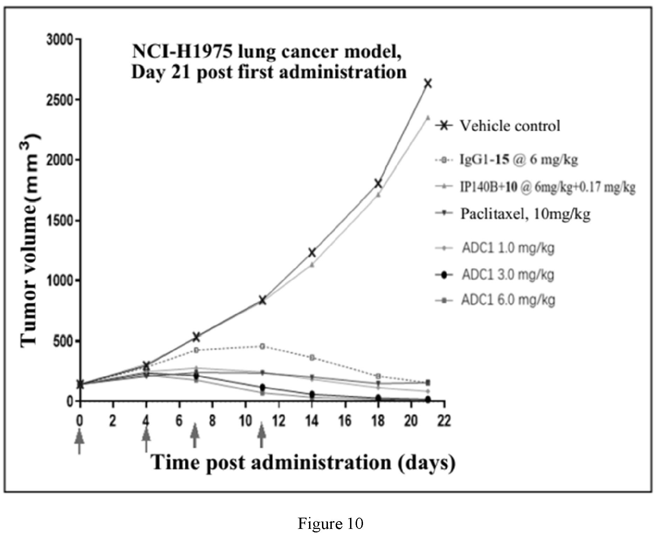

FIG. 10 shows a graph of tumor growth curves in an NCI-H1975 model in Test Example 1.

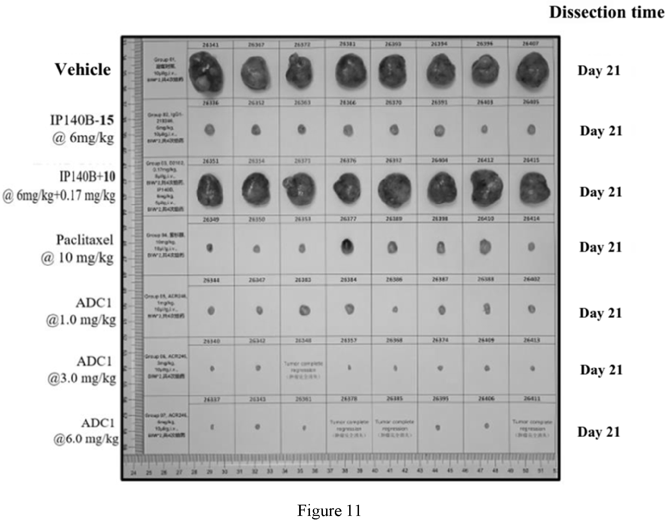

FIG. 11 shows images of tumors after dissection from the NCI-H1975 model in Test Example 1.

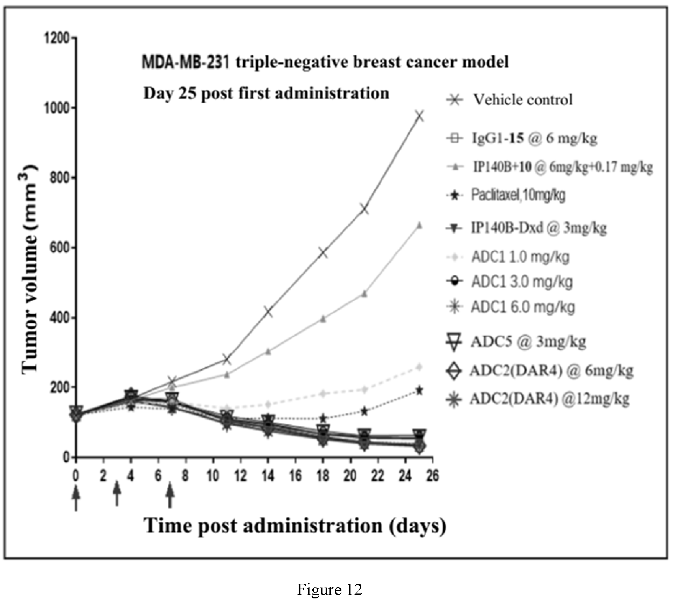

FIG. 12 shows a graph of tumor growth curves in an MDA-MB-231 model in Test Example 1.

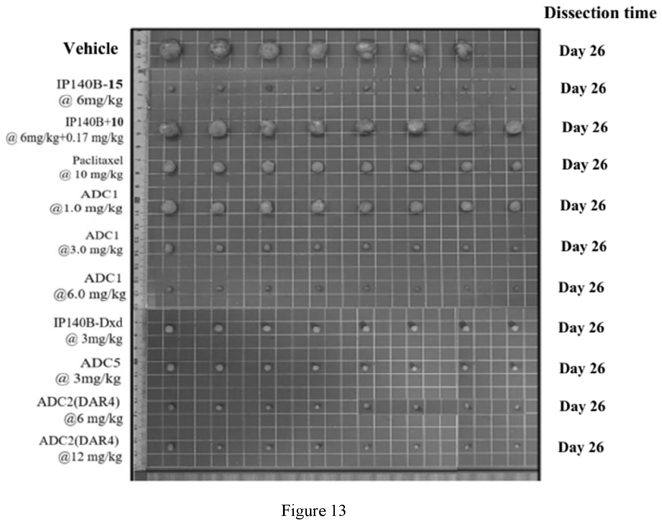

FIG. 13 shows images of tumors after dissection from the MDA-MB-231 model in Test Example 1.

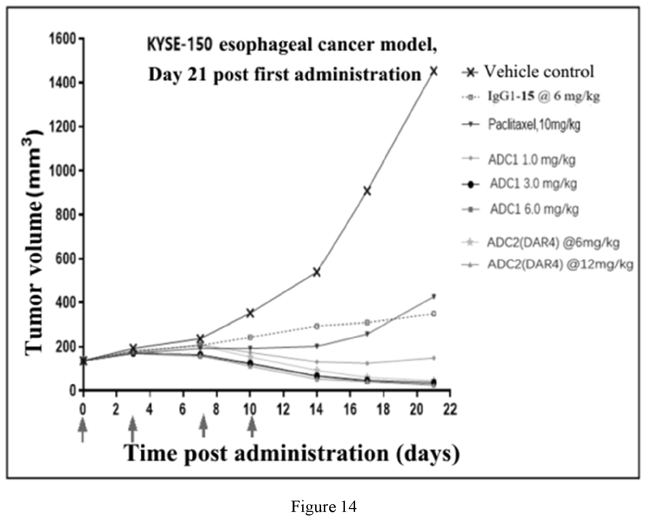

FIG. 14 shows a graph of tumor growth curves in a KYSE-150 model in Test Example 1.

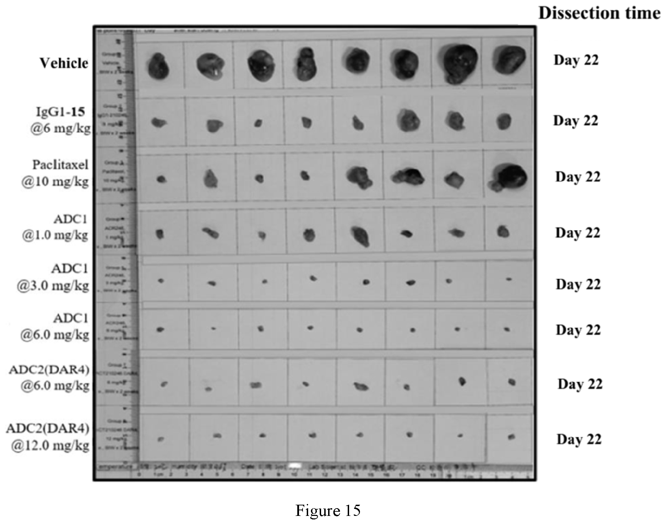

FIG. 15 shows images of tumors after dissection in the KYSE-150 model in Test Example 1.
FIG. 16 shows a graph of individual drug-time curves of ADC5-TAB in serum in cynomolgus monkeys after the cynomolgus monkeys received the 1st, 3rd, and 4th (Day 1, 38, and 52) intravenous injections with ADC5 at 22 and 35 mg/kg in Test Example 2.
FIG. 17 shows graphs of individual drug-time curves of toxin molecules in plasma in cynomolgus monkeys received the 3rd (Day 38, left) and 4th (Day 52, right) intravenous injections with ADC5 @ 35 mg/kg in Test Example 2.
FIG. 18 shows a graph of drug-time curves of ADC5 (ADC drug) and ADC5-TAB (total antibodies) in serum after the cynomolgus monkeys received intravenous injection with ADC5 in Test Example 2.
FIG. 19 shows a graph of drug-time curves of a payload (free toxin molecules) in serum after the cynomolgus monkeys received intravenous injection with ADC5 in Test Example 2.

### Detailed Description of Embodiments

### I. Definition

In the present disclosure, unless otherwise specified, scientific and technical nouns used herein have the meanings commonly understood by those skilled in the art. In addition, the relevant terms and laboratory operation steps used herein are all terms and conventional steps widely used in the corresponding fields. Moreover, for a better understanding of the present disclosure, definitions and explanations of related terms are provided below.

As used herein and unless otherwise specified, the term "about" or "approximately" means within plus or minus 10% of a given value or range. When integers are required, the term means within plus or minus 10% of the given value or range, rounded up or down to the nearest integer.

The description relates to "some examples", "some implementations", or "some embodiments" herein, which describe subsets of all possible examples. However, it can be understood that "some examples" can be the same subset or different subsets of all possible examples and can be combined with each other without conflict.

As used herein and unless otherwise specified, the terms "comprising", "including", "having", and "containing", including grammatical equivalents thereof, should generally be understood to be open-ended and non-limiting, for example, not excluding other unrecited elements or steps.

As used herein, the term "alkyl" refers to a linear or branched, saturated aliphatic hydrocarbon group containing from 1 to 20 carbon atoms. The term "C₁₋₆ alkyl" refers to a linear or branched alkyl group having from 1 to 6 carbon atoms, more preferably C₁₋₄ alkyl, and most preferably C₁₋₃ alkyl. Specific examples of alkyl include, but are not limited to, methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, various branched isomers thereof, etc.

The term "alkoxy" refers to -O-(alkyl) and -O-(unsubstituted cycloalkyl), wherein the alkyl is defined as previously. Non-limiting examples of alkoxyl include: methoxy, ethoxy, propoxy, butoxy, cyclopropoxy, cyclobutoxy, cyclopentyloxy, and cyclohexoxy. The alkoxy can be optionally substituted or unsubstituted. When the alkoxy is substituted, the substituent is preferably one or more groups independently selected from alkyl, alkenyl, alkynyl, alkoxy, alkylthio, alkylamino, halogen, mercapto, hydrogen, nitro, chloro, cycloalkyl, heterocycloalkyl, aryl, heteroaryl, cycloalkoxy, heterocycloalkoxy, cycloalkylthio, heterocycloalkylthio, carboxyl, or carboxylate group. 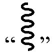 refers to a site to which a chemical bond is attached. It needs to be noted that the structural fragments described in the present disclosure (e.g., -L¹¹-L¹²-L¹³- or -L^{z}-L^{j}-), unless otherwise specified, represent corresponding groups connected in order from left to right. For example, when -L¹²- is C₁-C₂ alkylene-O-, it represents C₁-C₂ alkylene-O- linked to -L¹¹- on the left side and to -L¹³- on the right end.

It can be understood that in the antibody-drug conjugate of the present application, for example, in formula (I), the "-" between the drug-linker fragment and the antibody is intended to indicate the linking relationship between the antibody and the fragment and is not intended to be limited to one antibody linked to one drug-linker fragment. It is well known in the art that due to the presence of a plurality of interchain disulfide bonds on one antibody, one antibody can be linked to one or more drugs.

As used herein, the term "substituted" means that any one or more hydrogen atoms on a specific atom is replaced by a substituent, which can include heavy hydrogen and hydrogen variants, as long as the valence state of the specific atom is normal and the substituted compound is stable. When the substituent is oxo (i.e., =O), it means that two hydrogen atoms are replaced. Substitution with oxo does not occur on an aromatic group. The term "substituted optionally" or "optionally substituted" means that it may or may not be substituted, and unless otherwise specified, the type and number of substituents may be arbitrary, provided that it is chemically feasible.

Although the term "optionally substituted" does not appear, if any position on a ring (e.g., an aliphatic ring or aromatic ring) is substituted, it should be understood that the positions and number of substitutions are arbitrary, as long as they are chemically feasible.

As used herein, the term "antibody" is used in its broadest sense and specifically encompasses monoclonal antibodies (including full-length monoclonal antibodies), polyclonal antibodies, multispecific antibodies (e.g., bispecific antibodies), antibody fragments or synthetic polypeptides carrying one or more CDRs or derived from CDR sequences, as long as these polypeptides exhibit the desired biological activity. Antibodies (Abs) and immunoglobulins (Igs) are glycoproteins having identical structural characteristics. The "antibody" can also refer to immunoglobulins and immunoglobulin fragments, whether naturally occurring or partially or completely synthesized (e.g., by recombination), including any fragment which contains at least a partial variable region of an immunoglobulin molecule and retains the binding specificity of the full-length immunoglobulin. Therefore, an antibody includes any protein having a binding domain that is homologous or substantially homologous to an antigen-binding domain of an immunoglobulin (a binding site of an antibody). Antibodies include antibody fragments, e.g., anti-tumor stem cell antibody fragments. As used herein, therefore, the term "antibody" includes synthetic antibodies, recombinantly produced antibodies, multispecific antibodies (e.g., bispecific antibodies), human antibodies, non-human antibodies, humanized antibodies, chimeric antibodies, intracellular antibodies, and antibody fragments, for example, but not limited to, Fab fragments, Fab' fragments, F(ab')2 fragments, Fv fragments, disulfide-linked Fv(dsFv), Fd fragments, Fd' fragments, single-chain Fv (scFv), single-chain Fab (scFab), diabodies, anti-idiotypic (anti-Id) antibodies, or antigen-binding fragments of any of the foregoing antibodies. The antibodies provided herein include members of any immunoglobulin types (e.g., IgG, IgM, IgD, IgE, IgA, and IgY), any categories (e.g., IgG1, IgG2, IgG3, IgG4, IgA1, and IgA2), or any subclasses (e.g., IgG2a and IgG2b) (the terms "type" and "class", as well as "subtype" and "subclass", are used interchangeably herein). Natural or wild-type (i.e., derived from members of a non-artificially manipulated population) antibodies and immunoglobulins are typically heterotetrameric glycoproteins of about 150,000 Daltons, which are composed of two identical light chains (L) and two identical heavy chains (H). Each heavy chain has a variable domain (VH) at one end, followed by a plurality of constant domains. Each light chain has a variable domain (VL) at one end and a constant domain at the other end. The so-called "non-artificially manipulated" means not undergoing a treatment intended to cause it to contain or express a foreign antigen-binding molecule. Wild-type may refer to the most prevalent allele or species found in a population, or to antibodies derived from non-manipulated animals, as opposed to alleles or polymorphisms, or variants or derivatives obtained by using some forms of manipulation, e.g., mutagenesis or the use of recombinant approaches, to alter the amino acids of the antigen-binding molecule.

As used herein, the term "drug-to-antibody ratio (DAR)" refers to the average number of payload molecules (anti-tumor compounds or drugs) attached to a single monoclonal antibody.

As used herein, the term "pharmaceutically acceptable excipient" refers to a nontoxic, inert, solid or semi-solid material or liquid filling agent, diluent, encapsulating material, auxiliary formulating agent, or any type of excipient, which is compatible with patients, preferably mammals, more preferably humans, and which is suitable for delivering the active agent to the target of interest without compromising the activity of the agent. The pharmaceutically acceptable carriers and excipients in the present disclosure can be better compatible with the active ingredient, enabling the prepared pharmaceutical formulation to have dissolution, storage stability, impurity content, etc., which meet standards.

Typically, the antibody-drug conjugate of the present disclosure can be formed into suitable dosage forms with one or more pharmaceutically acceptable carriers for administration. These dosage forms are suitable for oral, rectal, topical, intraoral, and other parenteral (e.g., subcutaneous, intramuscular, and intravenous) routes of administration. For instance, dosage forms suitable for other parenteral routes of administration include injections, etc. The above dosage forms can be prepared from the active ingredient of the present disclosure together with one or more carriers or excipients by general pharmaceutical methods. The above carriers must be compatible with the active ingredient of the present disclosure or other excipients.

In embodiments of the present disclosure, the mode of administration of the pharmaceutical composition can include any one of oral, nebulized inhalation, rectal, nasal, buccal, topical, and parenteral routes of administration, such as subcutaneous, intravenous, intramuscular, intraperitoneal, intrathecal, intraventricular, intrasternal, and intracranial injection or infusion, or administration by means of an implanted reservoir. For oral administration, the compound of the present disclosure can be prepared into any orally acceptable formulation form, including but not limited to tablets, capsules, aqueous solutions, or aqueous suspensions. Carriers used for tablets generally include lactose and corn starch, and lubricants such as magnesium stearate may also be additionally added. Diluents used for capsule formulations generally include lactose and dried corn starch. Aqueous suspension formulations are generally used by mixing the active ingredient with suitable emulsifying agents and suspending agents. If needed, sweetening agents, flavoring agents, or coloring agents may also be added to the above oral formulation forms. The compound of the present disclosure may also be administered in the form of sterile injectable formulations, including sterile injectable aqueous or oily suspensions or sterile injectable solutions. Carriers and solvents that can be used include water, Ringer's solution, and an isotonic sodium chloride solution. Additionally, a sterile non-volatile oil may also be used as a solvent or suspending medium, and is, for example, monoglycerides or diglycerides.

The pharmaceutical composition of the present disclosure is formulated, dosed, and administered in a manner conforming to medical practice norms. The "therapeutically effective dose" of the active ingredient of the present disclosure is determined by the specific condition to be treated, the individual being treated, the cause of the condition, the target for the drug, the mode of administration, and other factors.

As used herein, the term "therapeutically effective amount" refers to an amount that is capable of eliciting a functionality or activity in a patient (e.g., a human and/or an animal) and is acceptable to the human and/or animal.

As used herein, "patient" refers to an animal, preferably a mammal, more preferably a human.

As used herein, "treatment" refers to alleviating, delaying the progression of, attenuating, preventing, or maintaining an existing disease or condition (e.g., cancer). "Treatment" also includes curing one or more symptoms of the disease or condition, preventing the development thereof, or reducing it to a certain extent.

### II. Examples

In order to make the object, technical solution, and advantages of the present disclosure clearer, the present disclosure will be further described in detail below. The described examples should not be regarded as limitations to the present disclosure, and all other examples obtained by those of ordinary skill in the art without creative efforts fall within the scope of protection of the present disclosure.

Before further detailed explanation of the examples of the present disclosure, the nouns and terms involved in the examples of the present disclosure will be explained. The nouns and terms involved in the examples of the present disclosure are applicable to the following explanations.

The raw materials and apparatuses used in the specific implementations of the present disclosure are all known products and obtained by purchasing commercially available products.

### Abbreviation:

Fmoc: 9-fluorenylmethoxycarbonyl; DCM: dichloromethane; DMF: N,N-dimethylformamide; THF: tetrahydrofuran; HOBT: 1-hydroxybenzotriazole; EDCI: 1-ethyl-(3-dimethylaminopropyl)carbodiimide hydrochloride; DIPEA: diisopropylethylamine; TFA: trifluoroacetic acid; HATU: 2-(7-azabenzotriazole)-N,N,N',N'-tetramethyluronium hexafluorophosphate; DIC: N,N'-diisopropylcarbodiimide; NHS: N-hydroxysuccinimide; DBU: 1,8-diazabicyclo[5.4.0]undec-7-ene; and DMA: N,N-dimethylaniline. Val: valine, the structural formula of which is 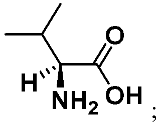 Ala: alanine, the structural formula of which is 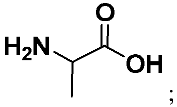 Gly: glycine, the structural formula of which is 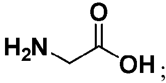 Phe: phenylalanine, the structural formula of which is 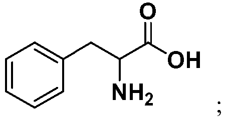 and Leu: leucine, the structural formula of which is 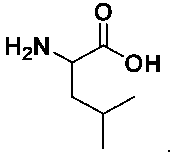 The peptide residue L^{P} can be obtained by a well-known amino acid condensation method in the art, and this is not particularly limited in the present disclosure.

In the present disclosure, the antibody and the linker L^{j} used for linking the antibody can be linked by a method well known in the art. For example, when the L^{j} structure is 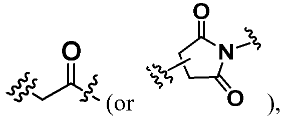 the L^{j} structure can be obtained by reacting 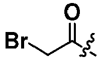 (or 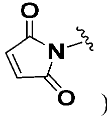 with a reactive group such as a thiol group on the antibody for connection, and this is not particularly limited in the present disclosure.

The IgG1 monoclonal antibody (Human IgG1, kappa isotype Control) in Test Example 1 was purchased from Sino-Biological under article number: HG1K.

In this example, the preparation of ADC used, without limitation, HS627, anti-IP140B antibody, and anti-Nectin-4 antibody. The heavy chain amino acid sequence of the anti-HS627 antibody was as follows (SEQ ID NO: 1):

EVQLVESGGGLVQPGGSLRLSCAASGFTFTDYTMDWVRQAPGKGLEWVADVNP
NSGGSIYNQRFKGRFTLSVDRSKNTLYLQMNSLRAEDTAVYYCARNLGPSFYFD
YWGQGTLVTVSSASTKGPSVFPLAPSSKSTSGGTAALGCLVKDYFPEPVTVSWN
SGALTSGVHTFPAVLQSSGLYSLSSVVTVPSSSLGTQTYICNVNHKPSNTKVDKK
VEPKSCDKTHTCPPCPAPELLGGPSVFLFPPKPKDTLMISRTPEVTCVVVDVSHED

PEVKFNWYVDGVEVHNAKTKPREEQYNSTYRVVSVLTVLHQDWLNGKEYKCK
VSNKALPAPIEKTISKAKGQPREPQVYTLPPSREEMTKNQVSLTCLVKGFYPSDIA
VEWESNGQPENNYKTTPPVLDSDGSFFLYSKLTVDKSRWQQGNVFSCSVMHEA
LHNHYTQKSLSLSPG

The light chain amino acid sequence thereof was as follows (SEQ ID NO: 2):

DIQMTQSPSSLSASVGDRVTITCKASQDVSIGVAWYQQKPGKAPKLLIYSASYRY
TGVPSRFSGSGSGTDFTLTISSLQPEDFATYYCQQYYIYPYTFGQGTKVEIKRTVA
APSVFIFPPSDEQLKSGTASVVCLLNNFYPREAKVQWKVDNALQSGNSQESVTEQ
DSKDSTYSLSSTLTLSKADYEKHKVYACEVTHQGLSSPVTKSFNRGEC

The heavy chain amino acid sequence of the anti-IP140B antibody was as follows (SEQ ID NO: 3):

QVQLVQSGGGLVQPGGSLRLSCAASGFTFSSYEMNWVRQAPGKGLEWVSYISSS
GSTIYYADSVKGRFTISRDNAKNSLYLQMNSLRAEDTAVYYCAREMQFGWELL
GAFDIWGQGTMVTVSSASTKGPSVFPLAPSSKSTSGGTAALGCLVKDYFPEPVTV
SWNSGALTSGVHTFPAVLQSSGLYSLSSVVTVPSSSLGTQTYICNVNHKPSNTKV
DKRVEPKSCDKTHTCPPCPAPELLGGPSVFLFPPKPKDTLMISRTPEVTCVVVDVS
HEDPEVKFNWYVDGVEVHNAKTKPREEQYNSTYRVVSVLTVLHQDWLNGKEY
KCKVSNKALPAPIEKTISKAKGQPREPQVYTLPPSRDELTKNQVSLTCLVKGFYPS
DIAVEWESNGQPENNYKTTPPVLDSDGSFFLYSKLTVDKSRWQQGNVFSCSVMH
EALHNHYTQKSLSLSPGK

The light chain amino acid sequence thereof was as follows (SEQ ID NO: 4):

EIVLTQSPGTLSLSPGERATLSCRASQSVSSSYLAWYQQKPGQAPRLLIYGASSRA
TGIPDRFSGSGSGTDFTLTISRLEPEDFAVYYCQQYGSSFGQGTKLEIKRTVAAPS
VFIFPPSDEQLKSGTASVVCLLNNFYPREAKVQWKVDNALQSGNSQESVTEQDS
KDSTYSLSSTLTLSKADYEKHKVYACEVTHQGLSSPVTKSFNRGEC

The heavy chain amino acid sequence of the anti-Nectin-4 antibody was as follows (SEQ ID NO: 5):

EVQLVESGGGLVQPGGSLRLSCAASGFTFSSYNMNWVRQAPGKGLEWVSYISSS
SSTIYYADSVKGRFTISRDNAKNSLSLQMNSLRDEDTAVYYCARAYYYGMDVW
GQGTTVTVSSASTKGPSVFPLAPSSKSTSGGTAALGCLVKDYFPEPVTVSWNSGA

LTSGVHTFPAVLQSSGLYSLSSVVTVPSSSLGTQTYICNVNHKPSNTKVDKRVEP
KSCDKTHTCPPCPAPELLGGPSVFLFPPKPKDTLMISRTPEVTCVVVDVSHEDPEV
KFNWYVDGVEVHNAKTKPREEQYNSTYRVVSVLTVLHQDWLNGKEYKCKVSN
KALPAPIEKTISKAKGQPREPQVYTLPPSREEMTKNQVSLTCLVKGFYPSDIAVE
WESNGQPENNYKTTPPVLDSDGSFFLYSKLTVDKSRWQQGNVFSCSVMHEALH
NHYTQKSLSLSPGK

The light chain amino acid sequence thereof was as follows (SEQ ID NO: 6):

DIQMTQSPSSVSASVGDRVTITCRASQGISGWLAWYQQKPGKAPKFLIYAASTLQ
SGVPSRFSGSGSGTDFTLTISSLQPEDFATYYCQQANSFPPTFGGGTKVEIKRTVA
APSVFIFPPSDEQLKSGTASVVCLLNNFYPREAKVQWKVDNALQSGNSQESVTEQ
DSKDSTYSLSSTLTLSKADYEKHKVYACEVTHQGLSSPVTKSFNRGEC

Testing and calculation for DAR value: Based on the RP-HPLC-MS test results, the DAR value of ADC was analyzed using Waters Acquity UPLC I-Class/Xevo G2-XS QTOF instrument.

RP-HPLC parameter settings: chromatography column PLRP-S 1000A 5 µm and column temperature 70°C. Mobile phase A was a 0.1% formic acid aqueous solution, and mobile phase B was a 0.1% formic acid acetonitrile solution, with a flow rate of 0.2 mL/min. The gradient of the mobile phase was 20-50% B over 18 min; 50-95% B over 5 min; 95-20% B over 0.1 min; and 20-20% B over 6.9 min.

MS parameter settings: capillary voltage 2.50 kV, cone voltage 100 V, mass analysis range m/z: 200 to 4000, MSE collision energy 20 to 45 eV, ion source temperature 120°C, atomization temperature 500°C, atomization flow rate 1000 L/hr, and internal standard leucine enkephalin. A test article was diluted to 1 mg/mL with a sample buffer, followed by the addition of TCEP having a final concentration of 50 mmol/L. The mixture was incubated at 37°C for 20 min, and 5 µL was injected. Light chain peaks were identified, the percentage of the peak areas was calculated, and the total peak area was 100. Similarly, heavy chain peaks were identified, the percentage of the peak areas was calculated, and the total peak area was 100. The weighted peak areas of the heavy and light chains were respectively calculated by multiplying the respective peak area percentages by the corresponding drug loads. The calculation formula of DAR value was: DAR = 2* (Σ weighted peak areas of light chains + Σ weighted peak areas of heavy chains) / 100. Example 1: Synthesis of 2-(((tert-butoxycarbonyl)(methyl)amino)oxy)acetic acid (2)

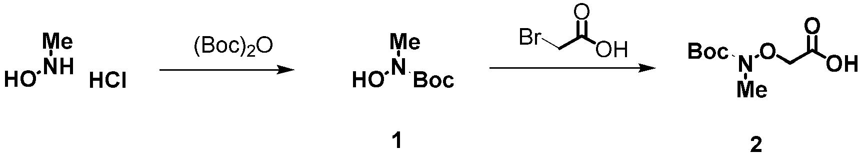

Step 1: To a 300 mL three-necked flask were successively added N-methylhydroxylamine hydrochloride (20 g, 239.5 mmol), water (60 mL), and THF (60 mL). The mixture was cooled to 0°C under ice bath condition. Sodium bicarbonate (20.1 g, 239.3 mmol) was added, followed by slow dropwise addition of di-tert-butyl dicarbonate (47.0 g, 215.3 mmol). The mixture was stirred at 0°C for 1 h and reacted at room temperature for 3 h. The reaction mixture was extracted with ethyl acetate (2 x 50 mL). The organic phases were combined, washed with a saturated aqueous sodium chloride solution (50 mL), 0.5 N dilute hydrochloric acid (30 mL), and then a saturated aqueous sodium chloride solution (50 mL), dried over anhydrous magnesium sulfate, filtered, and concentrated to give Compound 1 as a light yellow oily liquid (33 g, yield 93.7%, HPLC purity 98%); ¹H NMR (500 MHz, DMSO-*d*₆) δ 9.25 (s, 1H), 3.00 (s, 3H), 1.40 (s, 9H).

Step 2: Under ice bath cooling condition, to a 300 mL three-necked flask were added a 1.5 N NaOH aqueous solution (100 mL) and Compound 1 (10 g, 67.9 mmol), followed by the addition of a solution of bromoacetic acid (9.4 g, 67.7 mmol) in water (50 mL). The mixture was stirred at 0°C for 0.5 h and extracted with ethyl acetate (2 x 50 mL). The aqueous phases were adjusted to pH = 2 with 1N HCl, extracted with dichloromethane (3 x 50 mL), washed with a saturated aqueous sodium chloride solution, dried over anhydrous MgSO₄, filtered, and concentrated to give Compound 2 as a light yellow oily liquid (12 g, yield 86.1%, HPLC purity 95%); ¹H NMR (500 MHz, DMSO-*d*₆) δ 12.42 (s, 1H), 4.37 (s, 2H), 3.09 (s, 3H), 1.43 (d, *J =* 5.8 Hz, 9H); LCMS: [M-1]⁺ 204.12 (theoretical value: 205.10).

### Example 2: Synthesis of (S)-N-(chloromethyl)-2-(1,3-dioxoisoindolin-2-yl)propanamide (4)

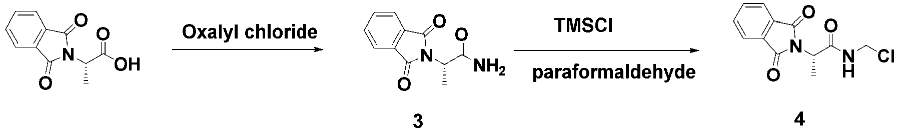

Step 1: To a 500 mL single-necked flask were added phthaloylalanine (5 g, 22.8 mmol), DCM (175 mL), and DMF (100 µL). The mixture was cooled to 0°C under ice bath condition. Oxalyl chloride (5.79 g, 45.6 mmol) was dropwise added under argon protection. The mixture was stirred at 0°C for 1.5 h and concentrated. The crude product was dissolved in DCM (90 mL) and cooled to 0°C. A 7M ammonia methanol solution (9.78 mL, 69.1 mmol) was dropwise added and reacted at room temperature under stirring for 2 h. n-Hexane was added. The mixture was filtered and dried to give Compound 3 (4.5 g, yield 92%, HPLC 97%); ¹H NMR (600 MHz, DMSO-*d*₆) δ 7.84 (d, J=4.9 Hz, 3H), 7.63 (s, 1H), 7.45 (s, 1H), 7.19 (d, J=10.6 Hz, 1H), 4.69 (q, J=7.2 Hz, 1H), 1.56 (d, J=7.3 Hz, 3H); LCMS: [M+1]⁺ 219.05 (theoretical value: 218.07).

Step 2: Compound 3 (1g, 4.5 mmol) was dissolved in DCM (10 mL). TMS-Cl (10 mL) and paraformaldehyde (550.4 mg, 18 mmol) were added. The tube was sealed, heated to 40°C, and reacted for 1.5 h. The reaction mixture was cooled, then filtered, and concentrated to give Compound 4 (1.22 g, yield 100%, HPLC purity 96%); ¹H NMR (500 MHz, CDCl₃) δ 7.85 (dd, *J=5.4,* 3.1 Hz, 2H), 7.78-7.72 (m, 2H), 7.19 (s, 1H), 5.23-5.13 (m, 2H), 5.03-4.74 (m, 2H), 1.69 (d, J=7.3 Hz, 3H); LCMS: [M+1]⁺ 266.98 (theoretical value: 266.05).

### Example 3: Synthesis of Fmoc-PEG₂-Gly-Val (8)

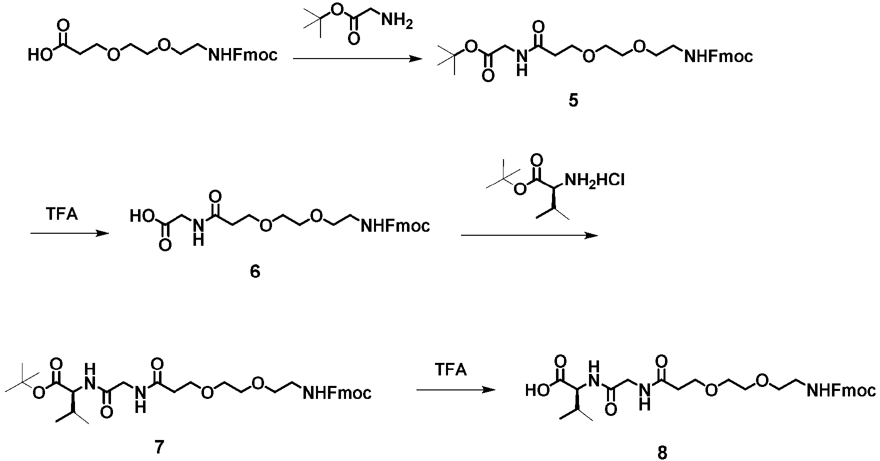

Step 1: To a 1000 mL single-necked flask was added dichloromethane (500 mL), followed by the addition of successively Fmoc-NH-PEG₂-CH₂CH₂-COOH (30 g, 75 mmol), HOBT (12.15 g, 90 mmol), EDCI (17.25 g, 90 mmol), tert-butyl glycinate (10.32 g, 78.75 mmol), and DIPEA (10.16 g, 90 mmol) under stirring. The mixture was reacted under stirring at room temperature for 2 h, washed with a saturated aqueous sodium chloride solution, a saturated aqueous sodium bicarbonate solution, and 0.5M hydrochloric acid, dried over anhydrous magnesium sulfate, filtered, and concentrated to give Compound 5 (36.2 g, yield 94%, HPLC 98%); ¹H NMR (600 MHz, CDCl₃) δ 7.78-7.67 (m, 2H), 7.60 (d, J=7.3 Hz, 2H), 7.41-7.34 (m, 2H), 7.34-7.24 (m, 2H), 6.78 (s, 1H), 5.60 (s, 1H), 4.45-4.15 (m, 2H), 3.90-4.00 (m, 2H), 3.80-3.33 (m, 10H), 2.90-2.42 (m, 3H), 1.44 (s, 9H); LCMS: [M+1]⁺ 513.14 (theoretical value: 512.25).

Step 2: To a 1000 mL single-necked flask were added dichloromethane (400 mL) and Compound 5 (36.2 g, 70 mmol). After the mixture was uniformly stirred, trifluoroacetic acid (100 mL) was dropwise added. The mixture was reacted under stirring at room temperature overnight and concentrated to give Compound 6 (32.2 g, yield 100%, HPLC 97%); ¹H NMR (600 MHz, CDCl₃) δ 8.88 (s, 1H), 7.72 (d, J=7.4 Hz, 2H), 7.60-7.50 (m, 2H), 7.40-7.33 (m, 2H), 7.30-7.20 (m, 2H), 7.19 (s, 1H), 5.68 (s, 1H), 7.45-7.33 (m, 2H), 4.45-4.35 (m, 2H), 4.25-4.15 (m, 2H), 4.00 (d, J=4.8 Hz, 1H), 3.69 (t, J=5.7 Hz, 2H), 3.60-3.20 (m, 8H), 2.50 (m, 2H); LCMS: [M+1]⁺ 457.08 (theoretical value: 456.19).

Step 3: To a 1000 mL single-necked flask were added dichloromethane (300 mL), followed by the addition of successively Compound 6 (5 g, 10.87 mmol), HOBT (1.76 g, 13.04 mmol), EDCI (2.5 g, 13.04 mmol), tert-butyl L-valinate hydrochloride (1.91 g, 11 mmol), and DIPEA (2.84 g, 22 mmol) under stirring. The mixture was stirred at room temperature for 2 h, washed with a saturated aqueous sodium chloride solution, a saturated aqueous sodium bicarbonate solution, and 0.5M hydrochloric acid, dried over anhydrous magnesium sulfate, filtered, and concentrated to give Compound 7 (5.5 g, yield 82%, HPLC 97%); ¹H NMR (600 MHz, CDCl₃) δ 7.80-7.70 (m, 2H), 7.61 (d, J=7.4 Hz, 2H), 7.41-7.33 (m, 2H), 7.31-7.25 (m, 2H), 6.97 (d, J=8.6 Hz, 1H), 5.98 (t, *J=*5.4 Hz, 1H), 4.50-4.36 (m, 3H), 4.21 (t, J=7.0 Hz, 1H), 4.05-3.95 (m, 2H), 3.75-3.65 (m, 1H), 3.64-3.50 (m, 6H), 3.45-3.30 (m, 2H), 2.51 (s, 1H), 2.20-2.10 (m, 1H), 1.43 (s, 9H), 0.90 (m, 6H); LCMS: [M+1]⁺ 612.05 (theoretical value: 611.32).

Step 4: To a 1000 mL single-necked flask were added dichloromethane (50 mL) and 7 (5.5 g, 9 mmol), followed by the dropwise addition of trifluoroacetic acid (10 mL) under stirring. Stirring was continued overnight. The mixture was concentrated to give Compound 8 (4.99 g, yield 100%, HPLC 96%); ¹H NMR (600 MHz, CDCl₃) δ 9.41 (s, 1H), 7.72 (d, J=7.4 Hz, 2H), 7.64-7.53 (m, 3H), 7.36 (q, J=7.7 Hz, 2H), 7.28 (q, J=7.4 Hz, 2H), 5.90-5.84 (s, 1H), 5.27 (s, 0H), 4.52-4.33 (m, 3H), 4.25-4.13 (m, 1H),4.14-4.09 (m, 1H), 4.07-4.01 (m, 1H), 3.97 (m, 1H),3.70 (s, 2H),3.57 (m, 3H), 3.52 (t, *J=*5.3 Hz, 2H), 3.41-3.23 (m, 2H), 3.22 (s, 1H), 2.51 (q, *J=10.5,* 5.5 Hz, 2H), 2.17 (dt, J=13.4, 6.6 Hz, 1H), 0.91 (dd, *J*=18.1, 6.8 Hz, 5H); LCMS: [M+1]⁺ 556.14 (theoretical value: 555.26).

### Example 4: Synthesis of N-(bromoacetyl-PEG2-Gly-Val-Ala-ha-N(Me)-aminooxyacetyl)exatecan (15)

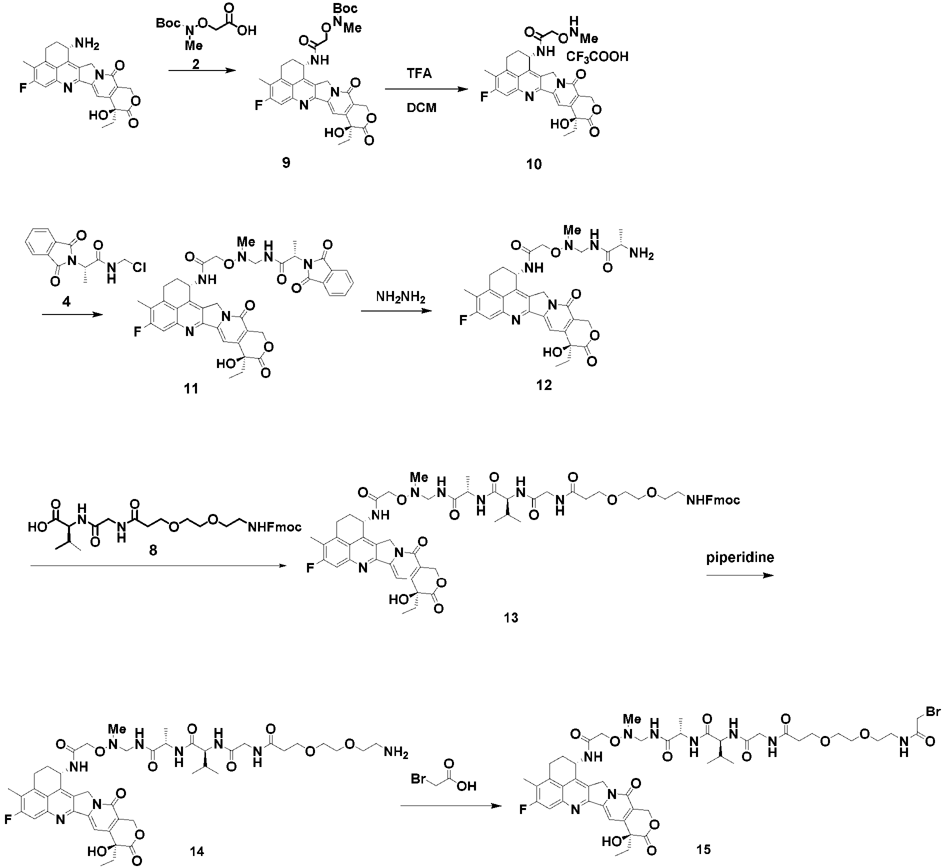

Step 1: Under argon protection, to a 100 mL three-necked flask were successively added Compound 2 (5.8 g, 28.3 mmol), DMF (50 mL), HATU (10.7 g, 28.2 mmol), exatecan mesylate (10 g, 18.8 mmol), and DIPEA (6.1 g, 47.2 mmol). The mixture was stirred to give a clear solution. The clear solution was heated to room temperature and reacted for 6 h. The reaction mixture was poured into 500 mL of ice water. After filtration, the filter cake was rinsed three times with water. The filter cake was dried in a forced-air drying oven (50°C, 24 h) to give Compound 9 (10.5 g, yield 90%, HPLC 89%); ¹H NMR (600 MHz, DMSO-*d*₆) δ 8.81 (d, J=8.6 Hz, 1H), 7.72 (d, J=10.7 Hz, 1H), 7.28-7.23 (m, 1H), 6.51 (d, J=1.9 Hz, 1H), 5.65-5.59 (m, 1H), 5.40 (d, J=3.8 Hz, 2H), 5.23-5.14 (m, 1H), 5.11 (d, J=19.0 Hz, 1H), 4.44-4.35 (m, 2H), 3.16 (d, J=6.6 Hz, 2H), 3.07 (s, 3H), 2.36-2.31 (m, 3H), 2.23 (dq, J=14.6, 5.0 Hz, 2H), 2.19 (s,2H), 1.85 (m, J=21.4, 7.3 Hz, 2H), 1.20 (s, 9H), 0.86 (t, J=7.3 Hz, 3H); LCMS: [M+1]⁺ 623.45 (theoretical value: 622.24).

Step 2: To a 250 mL three-necked flask were added DCM (100 mL) and Compound 9 (10.5 g, 16.9 mmol). The mixture was stirred and cooled to 0°C. TFA (25 mL) was dropwise added. The mixture was reacted under stirring at room temperature for 3 h and concentrated. Ethyl acetate/petroleum ether (1:1) was added. The mixture was filtered and dried to give Compound 10 (10 g, yield 93%, HPLC 87%); ¹H NMR (600 MHz, DMSO-*d*₆) δ 8.87 (d, *J*=8.6 Hz, 1H), 7.78 (d, *J*=10.8 Hz, 1H), 7.30 (s, 1H), 6.42 (s, 0H), 5.75 (s, 1H), 5.60 (dt, *J*=8.7, 4.4 Hz, 1H), 5.42 (s, 2H), 5.28 (s, 2H), 4.68-4.58 (m, 2H), 3.18 (p, *J=*6.3, 5.4 Hz, 2H), 2.84 (s, 3H), 2.39 (d, *J*=1.8 Hz, 3H), 2.24 (m, *J*=14.0*,* 4.8 Hz, 1H), 2.15 (m, *J=*10.4, 7.4, 4.4 Hz, 1H), 1.85 (m, *J*=21.4, 7.3 Hz, 2H), 0.86 (t, *J*=7.3 Hz, 3H); LCMS: [M+1]⁺ 523.38 (theoretical value: 522.19).

Step 3: To a 100 mL three-necked flask were added Compound 10 (6 g, 9.4 mmol), DMF (30 mL), and Compound 4 (3 g, 11.2 mmol). The mixture was stirred at 0-5°C. DIPEA (3.7 g, 28.6 mmol) was dropwise added. The mixture was reacted under stirring at room temperature for 1.5 h. Methyl tert-butyl ether (300 mL) was added. The supernatant was removed. A black solid was dissolved with 50 mL of DCM. 15 g of silica gel was added and blended for purification by silica gel column chromatography to give Compound 11 (6.2 g, yield 87.4%, HPLC 92%); ¹H NMR (500 MHz, DMSO-*d*₆) δ 8.72-8.66 (m, 1H), 8.49 (d, *J*=8.8 Hz, 1H), 7.79-7.71 (m, 1H), 7.70 (s, 3H), 7.28 (s, 1H), 6.51 (s, 1H), 5.39 (d, *J*=17.2 Hz, 3H), 5.23-5.19 (s, 1H), 5.00 (s, 1H), 4.68 (q, *J*=7.3 Hz, 1H),4.18 (s, 3H), 4.12-4.01 (m, 1H), 3.18 (d, *J*=17.1 Hz, 1H), 3.07 (d, *J*=17.6 Hz, 1H), 2.37 (s, 3H), 2.00-1.92 (m, 2H), 1.87-1.80 (m, 2H), 1.29 (m, *J*=7.2 Hz, 3H), 1.25-1.05 (m, 1H), 0.84 (q, *J*=13.5, 7.5 Hz, 3H); LCMS: [M+1]⁺ 753.12 (theoretical value: 752.26).

Step 4: Compound 11 (0.1 g, 0.13 mmol) was dissolved in MeOH (5 mL). Hydrazine hydrate (39.9 mg, 0.79 mmol) was added. The tube was sealed, heated to 60°C, and reacted for 2 h. The reaction mixture was cooled. The reaction liquid was poured into a saturated aqueous sodium chloride solution (100 mL), extracted with ethyl acetate, and concentrated to give Compound 12 (0.07 g, yield 77%, HPLC 95%); ¹H NMR (500 MHz, DMSO-*d*₆) δ 8.91 (t, *J*=6.0 Hz, 1H), 8.58 (d, *J*=8.9 Hz, 1H), 8.17 (s, 3H), 7.66 (d, *J*=10.8 Hz, 1H), 7.27 (s, 1H), 6.52 (s, 1H), 5.58 (td, *J*=8.3, 7.9, 4.8 Hz, 1H), 5.39 (s, 2H), 5.19 (d, *J*=19.0 Hz, 1H), 5.01 (d, *J*=18.9 Hz, 1H), 4.35 (dd, *J*=13.2*,* 6.3 Hz, 1H), 4.25 (dd, *J*=12.1, 4.7 Hz, 1H), 4.20 (d, *J*=2.3 Hz, 2H), 3.97-3.88 (m, 1H), 3.18 (dt, *J*=17.1, 5.9 Hz, 1H), 3.13-3.03 (m, 1H), 2.53 (s, 3H), 2.31 (d, *J*=1.9 Hz, 3H), 2.26-2.09 (m, 2H), 1.93-1.77 (m, *J*=7.2 Hz, 2H), 1.37 (d, *J*=6.9 Hz, 3H), 0.86 (t, *J*=7.3 Hz, 3H). LCMS: [M+1]⁺ 623.15 (theoretical value: 622.26).

Step 5: Compound 12 (70 mg, 0.112 mmol) were weighed and dissolved in DMF (2 mL). DIPEA (29 mg, 0.224 mmol) was added and stirred to give a clear solution. Compound 8 (62.16 mg, 0.112 mmol) and HATU (42.56 mg, 0.112 mmol) were successively added. The mixture was reacted under stirring at room temperature for 1 h. The reaction mixture was concentrated and purified by silica gel column chromatography to give Compound 13 (81 mg, yield 62%, HPLC 96%); ¹H NMR (500 MHz, DMSO-*d*₆) δ 8.50 (d, *J*=8.9 Hz, 1H), 8.24 (t, *J*=6.3 Hz, 1H), 8.11 (t, *J*=5.7 Hz, 1H), 7.99 (d, *J*=7.2 Hz, 1H), 7.86 (d, *J*=7.5 Hz, 2H), 7.74 (dd, J=9.8, 7.4 Hz, 2H), 7.67 (d, *J*=7.5 Hz, 2H), 7.39 (t, *J*=7.4 Hz, 2H), 7.35-7.26 (m, 4H), 6.52 (s, 1H), 5.75 (s, 2H), 5.59 (dt, *J*=9.2, 6.1 Hz, 1H), 5.41 (d, *J*=2.0 Hz, 2H), 5.25 (d, *J*=18.9 Hz, 1H), 5.09 (d, *J*=18.8 Hz, 1H), 4.33-3.99 (m, 8H), 3.70 (qd, *J*=16.6, 5.7 Hz, 2H), 3.57 (t, *J*=6.5 Hz, 2H), 3.46 (s, 4H), 3.39 (d, *J*=6.0 Hz, 2H), 3.26-3.06 (q, *J*=6.1 Hz, 5H), 2.48 (d, *J*=2.4 Hz, 3H), 2.41-2.29 (m, 5H), 2.17 (q, *J=6.5* Hz, 2H), 1.93-1.77 (m, 3H), 1.14 (d, *J*=7.1 Hz, 3H), 0.86 (t, *J*=7.3 Hz, 3H), 0.82-0.66 (m, 6H); LCMS: [M+1]⁺ 1160.24 (theoretical value: 1159.50).

Step 6: To a 10 mL single-necked flask was added Compound 13 (81 mg, 0.069 mg), which was dissolved with DMF (1 mL). Piperidine (100 µL) was added and reacted at room temperature for 1 h. Methyl tert-butyl ether (20 mL) was added. The mixture was centrifuged. The supernatant was removed. After drying, Compound 14 (32 mg, yield 49%, HPLC 94%) was obtained; ¹H NMR (500 MHz, DMSO-*d*₆) δ 8.53 (d, *J*=8.9 Hz, 1H), 8.28 (t, J=6.3 Hz, 1H), 8.13 (t, *J*=5.7 Hz, 1H), 8.01 (d, *J*=7.2 Hz, 1H), 7.77 (t, *J*=10.2 Hz, 5H), 7.30 (s, 1H), 6.53 (s, 1H), 5.60 (dt, *J*=8.9, 6.0 Hz, 1H), 5.41 (s, 2H), 5.27 (d, *J*=18.9 Hz, 1H), 5.13 (d, *J*=19.0 Hz, 1H), 4.28 (dd, *J*=13.2, 6.7 Hz, 1H), 4.24-4.12 (m, 3H), 4.12-4.03 (m, 2H), 3.79-3.64 (m, 2H), 3.63-3.48 (m, 8H), 3.27-3.08 (m, 2H), 2.97 (h, *J*=5.7 Hz, 2H), 2.47 (s, 3H), 2.37 (dd, *J*=7.8, 4.3 Hz, 5H), 2.18 (q, *J*=6.4 Hz, 2H), 1.85 (qq, *J*=14.0, 7.2 Hz, 3H), 1.15 (d, *J*=7.1 Hz, 3H), 0.86 (t, *J*=7.3 Hz, 3H), 0.71 (dd, *J*=13.3, 6.8 Hz, 6H); LCMS: [M+1]⁺ 938.02 (theoretical value: 937.43).

Step 7: To a 10 mL single-necked flask were successively added DCM (3mL), Compound 14 (32 mg, 0.034mmol), bromoacetic acid (9.5 mg, 0.068 mmol), and DIC (8.6 mg, 0.068 mmol). The mixture was reacted under stirring at room temperature for 90 min. The reaction mixture was concentrated and purified by silica gel column chromatography to give Compound 15 as a yellow solid (22 mg, yield 61%, HPLC 97%); ¹H NMR (500 MHz, DMSO-*d*₆) δ 8.49 (d, *J*=8.9 Hz, 1H), 8.33 (t, *J*=5.7 Hz, 1H), 8.24 (t, *J*=6.2 Hz, 1H), 8.11 (t, *J*=5.8 Hz, 1H), 7.98 (d, *J*=7.1 Hz, 1H), 7.75 (d, *J*=8.5 Hz, 1H), 7.70 (d, *J*=10.8 Hz, 1H), 7.28 (s, 1H), 5.64-5.54 (m, 1H), 5.40 (s, 2H), 5.23 (d, *J*=18.9 Hz, 1H), 5.04 (d, *J*=18.9 Hz, 1H), 4.33-3.97 (m, 6H), 3.85 (s, 2H), 3.70 (qd, *J*=16.5, 5.7 Hz, 3H), 3.57 (t, *J*=6.5 Hz,4H), 3.52-3.37 (m, 4H), 3.26-3.17 (m, 4H), 3.14-3.06 (m, 1H), 2.47 (s, 3H), 2.41-2.28 (m, 5H), 2.24-2.11 (m, *J*=6.9, 6.0 Hz, 2H), 1.84 (dh, *J*=20.8, 7.0 Hz, 3H), 1.14 (d, *J*=7.1 Hz, 3H), 0.85 (t, *J*=7.3 Hz, 3H), 0.70 (dd, *J*=11.4, 6.8 Hz, 6H); LCMS: [M+1]⁺ 1058.24 (theoretical value: 1057.36).

### Example 5: N-(maleimidoethylamino(N-carboxyethyl)-Gly-Ala-ha-N(Me)-aminooxyacetyl)exatecan (16)

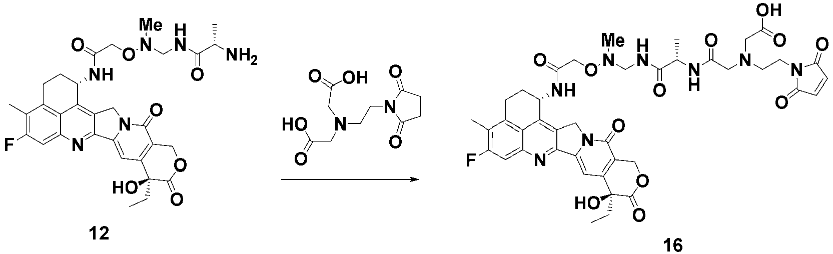

Step 1: To a single-necked flask containing 5 mL of anhydrous DMF was added Compound 12 (200 mg, 0.32 mmol). The mixture was stirred until complete dissolution was achieved. Another clean flask was taken, into which maleimidoethylamine-N,N-diacetic acid (226.1 mg, 0.64 mmol) and DIC (81.0 mg, 0.64 mmol) were weighed. The flask was shaken until the mixture was dissolved in a mixed solvent of dichloromethane (2 mL) and DMF (0.5 mL). A solution of Compound 12 in DMF was placed in an ice bath. A mixed solution of DIC and maleimidoethylamine-N,N-diacetic acid was added. The system was allowed to warm to room temperature naturally and stirred overnight. Dichloromethane was removed under reduced pressure. The product was wet-loaded and purified by reversed-phase column chromatography (ACN% = 25-40) to give Compound 16 as a white solid (100 mg, yield 36%); ¹H NMR (600 MHz, DMSO-*d*₆) δ 12.39 (s, 1H), 8.53 (d, J=8.9 Hz, 1H), 8.38 (t, J=6.2 Hz, 1H), 7.76 (dd, J=13.8, 8.9 Hz, 2H), 7.31 (s, 1H), 6.95 (s, 2H), 6.52 (s, 1H), 5.65-5.57 (m, 1H), 5.43 (s, 2H), 5.27 (d, J=19.0 Hz, 1H), 5.17 (d, J=18.8 Hz, 1H), 4.27-4.08 (m, 6H), 3.42 (t, J=6.3 Hz, 3H), 3.19-3.11 (m, 3H), 2.68 (t, *J=6.5* Hz, 2H), 2.48 (s, 4H), 2.39 (d, J=1.8 Hz, 4H), 2.19 (q, J=6.3 Hz, 2H), 1.86 (dp, *J*=21.4, 7.2 Hz, 3H), 1.13 (d, *J*=7.1 Hz, 3H), 0.87 (t,J=7.3 Hz, 4H); LC/MS: [M+1]⁺ 862.22 (theoretical value: 860.35).

### Example 6: N-(maleimidoethylamino(N-carboxyethyl)-Gly-PEG₂-Gly-Val-Ala-ha-N(Me)-aminooxyacetyl)exatecan (17)

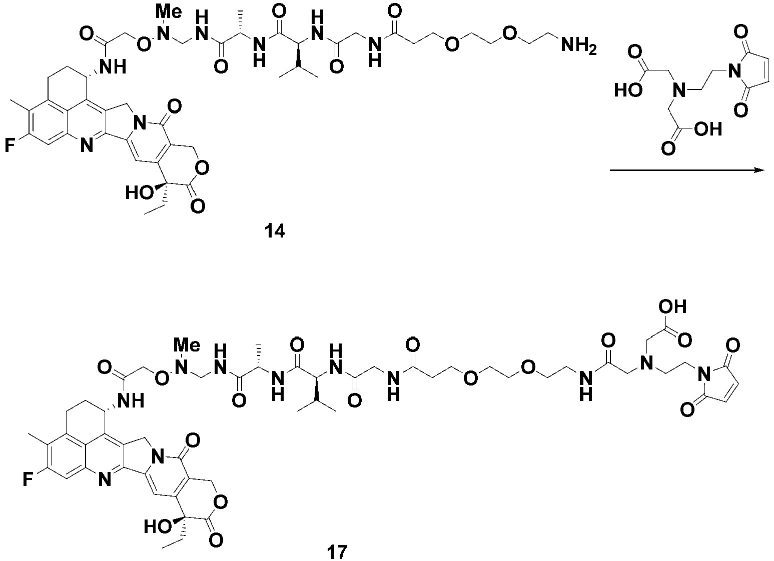

Step 1: To a single-necked flask containing 5 mL of anhydrous DMF was added Compound 14 (200 mg, 0.213 mmol). The mixture was stirred until complete dissolution was achieved. Another clean flask was taken, into which maleimidoethylamine-N,N-diacetic acid (151.89 mg, 0.43 mmol) and DIC (54.44 mg, 0.43 mmol) were weighed. The flask was shaken until the mixture was dissolved in a mixed solvent of dichloromethane (2 mL) and DMF (0.5 mL). A solution of Compound 14 in DMF was placed in an ice bath. A mixed solution of DIC and maleimidoethylamine-N,N-diacetic acid was added. The system was allowed to warm to room temperature naturally and stirred overnight. Dichloromethane was removed under reduced pressure. The product was wet-loaded and purified by reversed-phase column chromatography (ACN% = 25-35) to give Compound 1 as a white solid (84 mg, yield 33.5%); ¹H NMR (600 MHz, DMSO-*d*₆) δ 8.55 (d, *J=9.0* Hz, 1H), 8.33-8.16 (m, 2H), 8.08 (s, 1H), 7.80 (dd, J=22.2, 10.7 Hz, 2H), 7.31 (s, 1H), 6.99 (s, 1H), 6.52 (s, 1H), 5.61 (dd, J=9.2, 5.5 Hz, 1H), 5.42 (s, 2H), 5.29 (d, J=19.0 Hz, 1H), 5.18 (d, *J=18.7* Hz, 1H), 4.28 (dd, *J=*13.1, 6.7 Hz, 1H), 4.22-4.16 (m, 2H), 4.14 (dd, *J=12.2,* 5.2 Hz, 1H), 4.10-4.06 (m, 1H), 4.05-3.99 (m, 1H), 3.73 (dd, *J=16.5,* 5.7 Hz, 1H), 3.66 (dd, *J=16.6,* 5.8 Hz, 1H), 3.57 (t, J=6.5 Hz, 2H), 3.45 (d, *J=3.0* Hz, 6H), 3.38 (t, *J*=6.2 Hz, 5H), 3.25-3.12 (m, 8H), 2.70 (t, J=6.5 Hz, 1H), 2.47 (s, 4H), 2.39 (d, *J*=5.0 Hz, 3H), 2.35 (t, J=6.5 Hz, 2H), 2.19 (p, J=6.9, 5.8 Hz, 2H), 1.90-1.80 (m, 3H), 1.13 (d, J=7.1 Hz, 3H), 0.87 (t, J=7.3 Hz, 3H), 0.81 (t, J=6.5 Hz, 1H), 0.71 (dd, J=13.7, 6.7 Hz, 5H); LCMS: [M+1]⁺ 1176.45 (theoretical value: 1175.49).

### Example 7: Synthesis of N-(bromoacetyl-PEG₂-Gly-Val-Ala-ha-N(OMe)-aminoacetyl)exatecan (23)

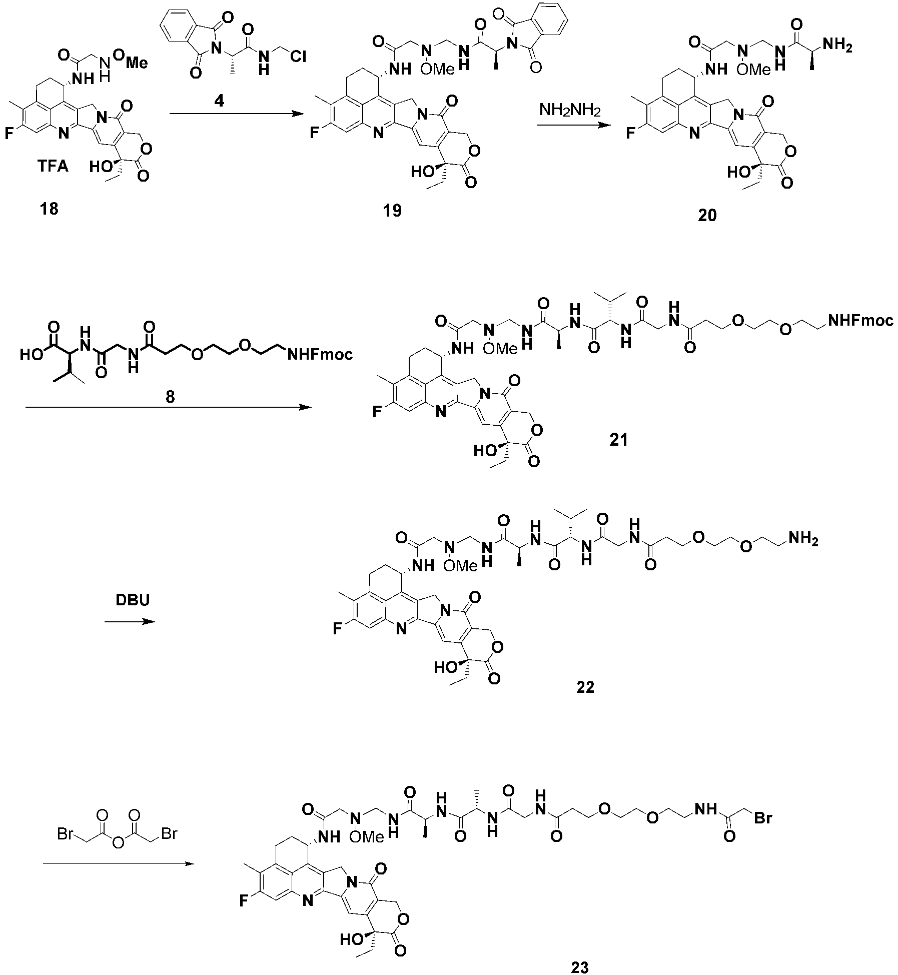

Step 1: To 1 mL of DMF were added exatecan mesylate (100 mg, 0.19 mmol) and TEA (25 mg, 0.24 mmol). The mixture was stirred to give a clear solution. Bromoacetic acid (40 mg, 0.28 mmol) and HATU (85 mg, 0.22 mmol) were added. The mixture was reacted under stirring at room temperature for 1 h. DMF was removed under reduced pressure. The resulting material was separated and purified by silica gel column chromatography to give intermediate exatecan-N-bromoacetamide (90 mg, yield 86%); LCMS: [M+1]⁺ 557.1 (theoretical value: 556.39).

Step 2: Exatecan-N-bromoacetamide was dissolved in DMF (1 mL). Methoxyamine hydrochloride (126 mg, 1.5 mmol) and TEA (182 mg, 1.8 mmol) were added. The mixture was reacted under stirring at room temperature for 1 h. DMF was removed under reduced pressure. The resulting material was separated and purified by silica gel column chromatography to give Compound 18 (80 mg, yield 86%); ¹H NMR (500 MHz, DMSO-*d*₆) δ 8.50-8.46 (m, 1H), 7.80 (d, J=10.8 Hz, 1H), 7.30 (d, J=1.9 Hz, 1H), 6.83 (td, J=6.2, 1.8 Hz, 1H), 6.52 (d, *J*=1.9 Hz, 1H), 5.61-5.55 (m, 1H), 5.42 (s, 2H), 5.22 (s, 2H), 4.01-3.87 (m, 1H), 3.42 (d, J=6.1 Hz, 3H), 3.17 (t, J=6.4 Hz, 2H), 2.40 (s, 3H), 2.22-2.11 (m, 2H), 1.86 (dp, J=21.1, 7.1 Hz, 2H), 0.90-0.83 (m, 3H); LCMS:[M+1]⁺ 523.20 (theoretical value: 522.19).

Step 3: DMF (12 mL) was added to a 50 mL three-necked flask. At 0-5°C, Compound 18 (1.68 g, 2.2 mmol), DIPEA (1.17 g, 9.1 mmol), and Compound 4 (1.75 g, 6.6 mmol) were successively added and reacted under stirring at 5-10°C for 2 h. Methyl tert-butyl ether (60 mL) was added. The supernatant was decanted to give Compound 19 (LCMS [M+H]⁺: 753.27; theoretical value: 752.26), which was dissolved in DCM (25 mL). 80% hydrazine hydrate (0.94 g, 15 mmol) was dropwise added under stirring. The mixture was stirred at room temperature overnight, concentrated, dissolved with DMF and water, and subjected to preparative liquid chromatography. After freeze drying, Compound 20 (1.08 g, combined yield 50% over two steps) was obtained; ¹H NMR (500 MHz, DMSO-*d*₆) δ 8.91 (t, J=6.0 Hz, 1H), 8.51 (d, *J*=8.7 Hz, 1H), 8.11 (s, 3H), 7.78 (d, J=10.9 Hz, 1H), 7.31 (s, 1H), 6.54 (s, 1H), 5.63-5.54 (m, 1H), 5.42 (s, 2H), 5.19 (s, 2H), 4.35-4.39 (m, 1H), 4.25-4.30 (m, 1H), 3.88 (s, 1H), 3.50 (s, 3H), 3.25-3.06 (m, 2H), 2.39 (s, 3H), 2.29-2.11 (m, 2H), 1.81-1.93 (m, 2H), 1.37 (d, J=7.0 Hz, 3H), 0.87 (t, J=7.3 Hz, 3H); LCMS(ESI)[M+H]⁺: 623.20 (theoretical value: 622.26).

Step 4: To a 25 mL single-necked flask were added Compound 8 (0.68 g, 1.22 mmol) and DCM (9 mL). The mixture was cooled to 0°C. EDCI (0.25 g, 1.30 mmol) and NHS (0.15 g, 1.52 mmol) were successively added. The mixture was reacted under stirring at room temperature for 2 h. DIPEA (0.19 g, 1.47 mmol) was dropwise added to the reaction liquid at 0°C under argon protection. A solution of Compound 20 (0.9 g, 1.25 mmol) in DMF (diluted with 5 mL of DMF) was slowly dropwise added to the reaction system. The mixture was reacted at room temperature overnight. DMF was removed under vacuum. The resulting material was purified by silica gel column chromatography to give Compound 21 (500 mg, yield 32%); ¹H NMR (500 MHz, DMSO-*d*₆) δ 8.50 (d, J=8.7 Hz, 1H), 8.39 (t, J=6.1 Hz, 1H), 8.13 (t, *J*=5.7 Hz, 1H), 8.09 (d, J=7.2 Hz, 1H), 7.91 (d, J=7.5 Hz, 2H), 7.80 (t, *J=10.4* Hz, 2H), 7.72 (d, J=7.4 Hz, 2H), 7.44 (t, J=7.4 Hz, 2H), 7.38-7.26 (m, 4H), 6.56 (s, 1H), 5.67-5.56 (m, 1H), 5.45 (s, 2H), 5.23 (s, 2H), 4.35-4.12 (m, 5H), 3.82-3.69 (m, 2H), 3.62 (t, J=6.4 Hz, 2H), 3.55-3.45 (m, 6H), 3.43 (t, *J=5.8* Hz, 2H), 3.22-3.12 (m, 4H), 2.45-2.35 (m, 5H), 2.32-2.12 (m, 2H), 1.95-1.83 (m, 3H), 1.34-1.19 (m, 8H), 0.90 (t, J=7.3 Hz, 3H), 0.74-0.80 (m, 6H); LCMS(ESI)[M+H]⁺: 1160.26 (theoretical value: 1159.50).

Step 5: Compound 21 (422 mg, 0.36 mmol) was added to DMF (2 mL) and dissolved under stirring. At 0°C, DBU (28 mg, 1.84 mmol) was dropwise added. The mixture was stirred at room temperature for 2 h. Methyl tert-butyl ether (21 mL) was added. The supernatant was removed to give Compound 22 (LCMS [M+H]⁺: 938.23; theoretical value: 937.43), which was dissolved in DMF (1.1 mL) and stirred. At 0°C, a solution of bromoacetic acid anhydride (0.95 g, 3.66 mmol) in DMF (1.9 mL) was dropwise added and stirred at room temperature for 2 h. Methyl tert-butyl ether (20 mL) was added. The supernatant was removed. The resulting viscous material was dissolved in DMF and purified by liquid chromatography, followed by freeze drying to obtain Compound 23 (100 mg, combined yield 26% over two steps); ¹H NMR (500 MHz, DMSO) δ 8.47 (d, *J=8.0* Hz, 1H), 8.39-8.24 (m, 2H), 8.16-8.01 (m, 2H), 7.77 (d, *J=10.0* Hz, 2H), 7.30 (s, 1H), 5.59 (s, 1H), 5.42 (s, 2H), 5.19 (s, 2H), 4.38-4.08 (m, 4H), 3.90-3.58 (m, 14H), 3.50-3.14 (m, 10H), 2.43-2.33 (m, 4H), 2.19 (s, 2H), 1.92-1.82 (m, 3H), 1.20 (d, J=6.6 Hz, 3H), 0.87 (t, J=6.7 Hz, 3H), 0.73 (dd, J=13.8, 6.3 Hz, 5H); ¹³C NMR (126 MHz, DMSO-*d*₆) δ 173.65, 172.90, 170.98, 170.88, 169.32, 168.70, 166.55, 163.10, 161.12, 157.16, 152.84, 150.43, 145.69, 140.84, 136.81, 126.10, 124.23, 124.08, 122.11, 119.59, 110.25, 97.14, 72.81, 69.97, 69.89, 69.19, 67.16, 65.71, 60.62, 59.30, 58.35, 57.74, 50.07, 48.67, 44.92, 42.42, 36.33, 31.07, 30.77, 29.92, 28.19, 19.45, 18.54, 18.34, 11.47, 11.43, 8.21; LCMS(ESI)[M+H]⁺: 1058.26 (theoretical value: 1057.36).

### Example 8: N-(m-ethylamino(N-carboxyethyl)-Gly-Ala-ha-N(OMe)-aminoacetyl)exatecan (24)

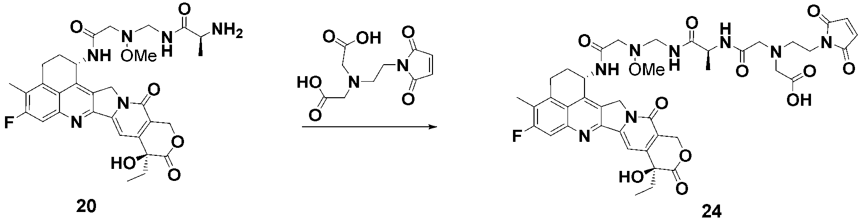

Step 1: To a 50 mL round-bottomed flask were added Compound 20 (80 mg, 0.129 mmol) and DMF (5 mL). The mixture was stirred until complete dissolution was achieved (Solution 1). Another 10 mL reaction flask was taken, to which were added maleimidoethylamino-N,N-diacetic acid (91 mg, 0.258 mmol), DIC (32.51 mg, 0.258 mmol), dichloromethane (4 mL), and DMF (2 mL). The mixture was fully shaken until dissolution was achieved (Solution 2). The solution of Compound 20 in DMF (Solution 1) was placed in an ice bath. Solution 2 was dropwise added to Solution 1 under stirring. The ice bath was removed, and the reaction system was allowed to warm to room temperature naturally and stirred overnight. Dichloromethane was removed under reduced pressure. The resulting material was purified by reversed-phase chromatography column (ACN% = 35%) to give Compound 24 as a yellow powder (32 mg, yield 28.8%); ¹H NMR (600 MHz, DMSO-*d*₆) δ 8.48 (d, J=8.9 Hz, 1H), 8.43 (t, *J*=6.3 Hz, 1H), 7.78 (d, J=10.8 Hz, 2H), 7.30 (s, 1H), 6.96 (s, 1H), 6.52 (s, 1H), 5.61-5.56 (m, 1H), 5.42 (s, 2H), 5.25-5.17 (m, 2H), 4.28-4.21 (m, 2H), 4.19-4.13 (m, 1H), 3.49-3.42 (m, 6H), 3.18 (t, *J=5.5* Hz, 5H), 2.70 (t, J=6.8 Hz, 2H), 2.39 (s, 3H), 2.22-2.13 (m, 2H), 1.85 (dq, J=21.6, 6.9 Hz, 2H), 1.21 (dd, J=32.6, 6.2 Hz, 5H), 0.87 (t, J=7.4 Hz, 4H); LCMS (ESI) [M+H]⁺ 862.05 (theoretical value 860.31).

### Example 9: Preparation of anti-IP140B antibody-drug conjugates ADC1 and ADC2 (1) Preparation of ADC1 (DAR 8)

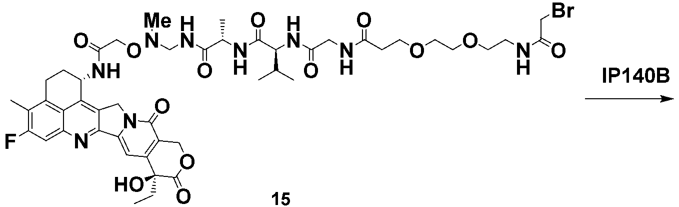

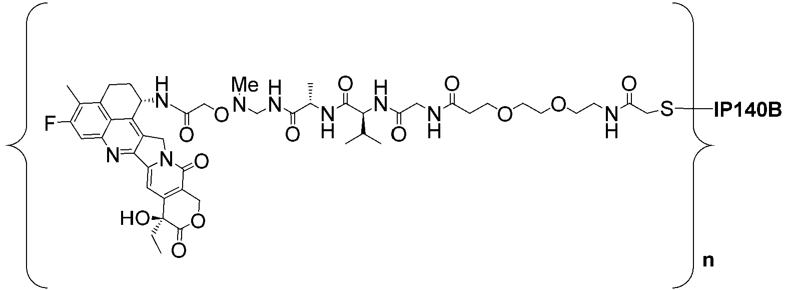

Anti-IP140B antibody (10.0 mg/mL, 10 mg, 0.066 mmol) was taken and adjusted to pH 7.2 with a 1M Na₂HPO₄ solution. A 0.1M disodium ethylenediaminetetraacetate solution (25 µL) was then added. A prepared TCEP·HCl (tris(2-carboxyethyl)phosphine hydrochloride) solution (10 mM, 0.04 mL) was added. The mixture was reacted on a rotary table at room temperature 25°C for 4 h.

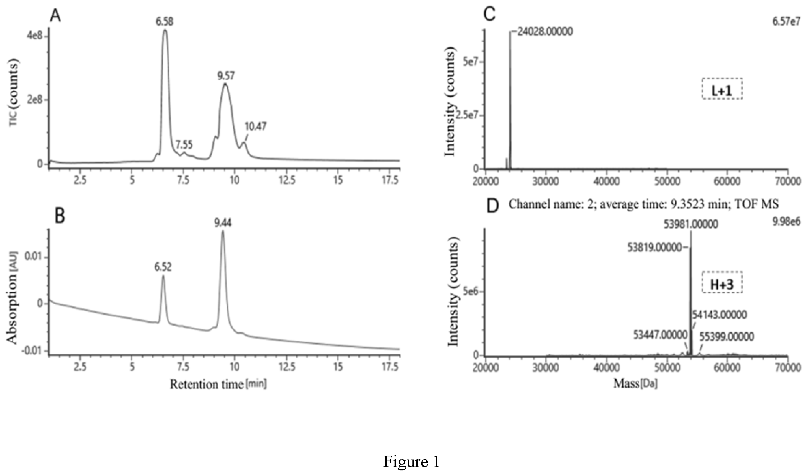

Compound 15 (0.99 mg, 0.93 mmol) was dissolved in 0.1 mL of DMA, added to the above solution system, uniformly mixed, and reacted on a rotary table at room temperature for 16 h. After the reaction was complete, small molecules were removed by using NAP-5 gel column (Cytiva), and the buffer was exchanged to a 20 mM histidine-histidine hydrochloride solution (pH = 6.2) to give the antibody-drug conjugate ADC1 (3.2 mg/mL, 2 mL). The RP-HPLC-MS test results thereof were shown in FIG. 1, wherein A and B were the HPLC results, and C and D were the MS results. The average value calculated based on RP-MS was n = 7.9. The MS results showed that one linker-payload was linked to the light chain (L) of the antibody, and three linker-payloads were linked to the heavy chain (H).

### (2) Preparation of ADC2 (DAR 4)

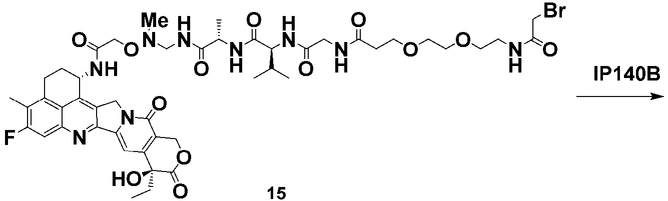

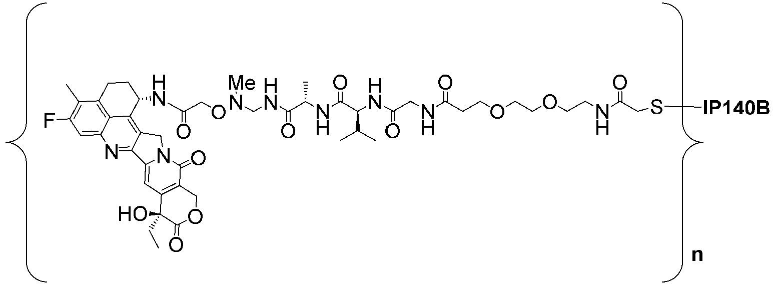

Anti-IP140B antibody (10.0 mg/mL, 10 mg, 0.066 mmol) was taken and adjusted to pH 7.2 with a 1M Na₂HPO₄ solution. A prepared TCEP·HCl solution (10 mM, 0.02 mL) was added. The mixture was reacted on a rotary table at 10°C for 2 h.

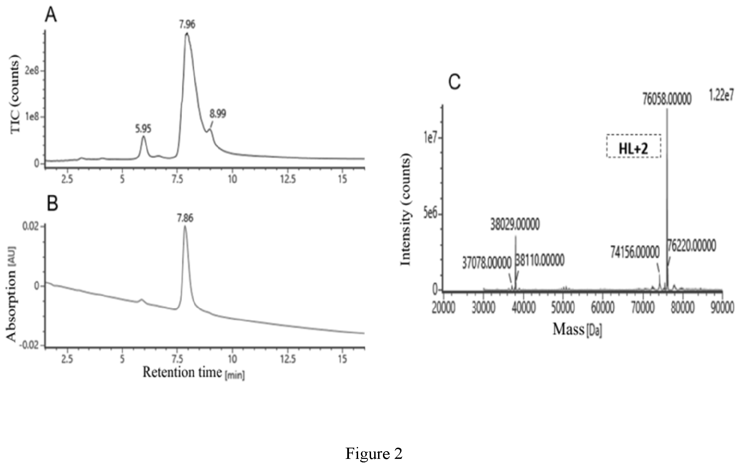

Compound 15 (0.59 mg, 0.56 mmol) was dissolved in 0.1 mL of DMA, added to the above solution system, uniformly mixed, and reacted on a rotary table at room temperature for 16 h. After the reaction was complete, small molecules were removed by using NAP-5 gel column (Cytiva), and the buffer was exchanged to a 20 mM histidine-histidine hydrochloride solution (pH = 6.2) to give the antibody-drug conjugate ADC2 (3.0 mg/ml, 2 mL). The RP-HPLC-MS test results thereof were shown in FIG. 2, wherein A and B were the HPLC results, and C was the MS result. The average value calculated based on RP-MS was n = 3.9. The MS results showed that two linker-payloads were linked to the light chain and heavy chain (HL) of the antibody.

### Example 10: Preparation of anti-HS627 antibody-drug conjugate ADC3

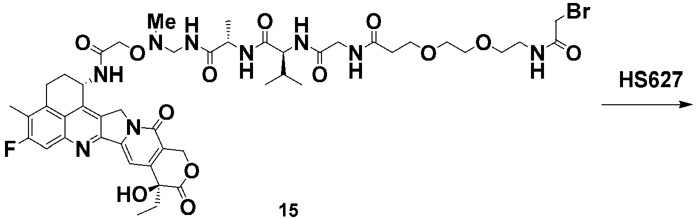

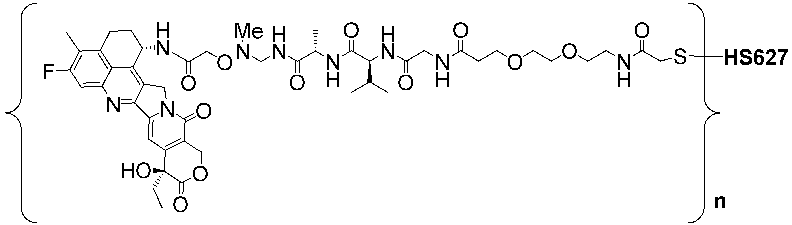

Anti-HS627 antibody (10.0 mg/mL, 10 mg, 0.066 mmol) was taken and adjusted to pH 7.2 with a 1M Na₂HPO₄ solution. A 0.1M disodium ethylenediaminetetraacetate solution (25 µL) was then added. A prepared TCEP·HCl (tris(2-carboxyethyl)phosphine hydrochloride) solution (10 mM, 0.04 mL) was added. The mixture was reacted on a rotary table at room temperature 25°C for 4 h.

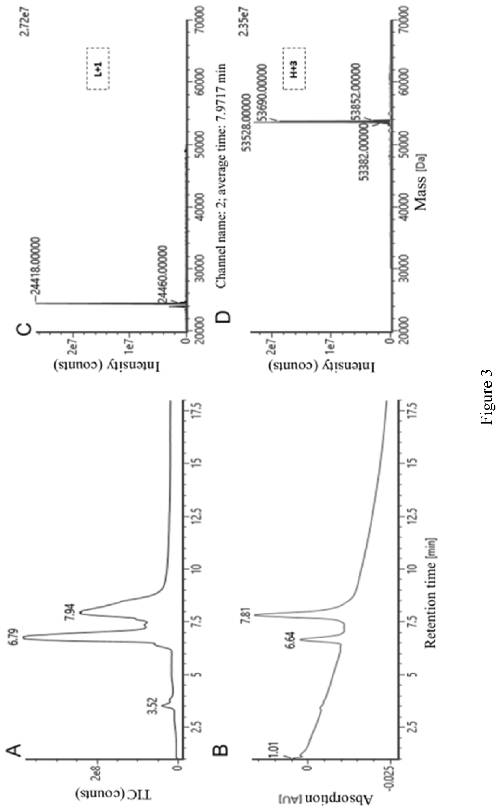

Compound 15 (0.99 mg, 0.93 mmol) was dissolved in 0.1 mL of DMA, added to the above solution system, uniformly mixed, and reacted on a rotary table at room temperature for 16 h. After the reaction was complete, small molecules were removed by using NAP-5 gel column (Cytiva), and the buffer was exchanged to a 20 mM histidine-histidine hydrochloride solution (pH = 6.2) to give the antibody-drug conjugate ADC3 (3.0 mg/ml, 2 mL).The RP-HPLC-MS test results thereof were shown in FIG. 3, wherein A and B were the HPLC results, and C and D were the MS results. The average value calculated based on RP-MS was n = 7.8. The MS results showed that one linker-payload was linked to the light chain (L), and three linker-payloads were linked to the heavy chain (H).

### Example 11: Preparation of anti-Nectin-4 antibody-drug conjugate ADC4

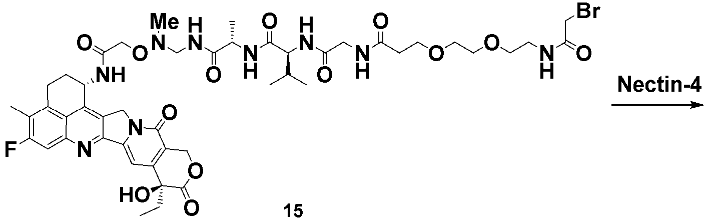

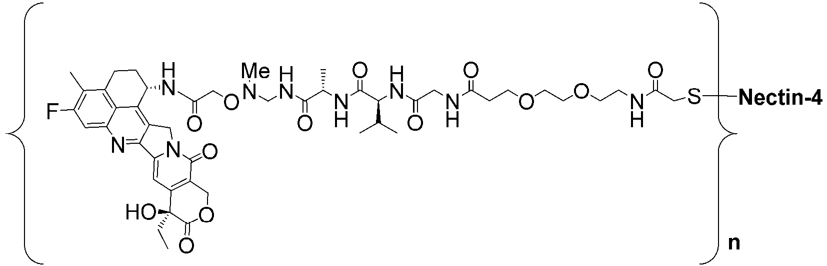

Anti-Nectin-4 antibody (10.0 mg/mL, 10 mg, 0.066 mmol) was taken and adjusted to pH 6.2 with a 1M Na₂HPO₄ solution. A 0.1M disodium ethylenediaminetetraacetate solution (25 µL) was then added. A prepared TCEP·HCl (tris(2-carboxyethyl)phosphine hydrochloride) solution (10 mM, 0.04 mL) was added. The mixture was reacted on a rotary table at room temperature 25°C for 4 h.

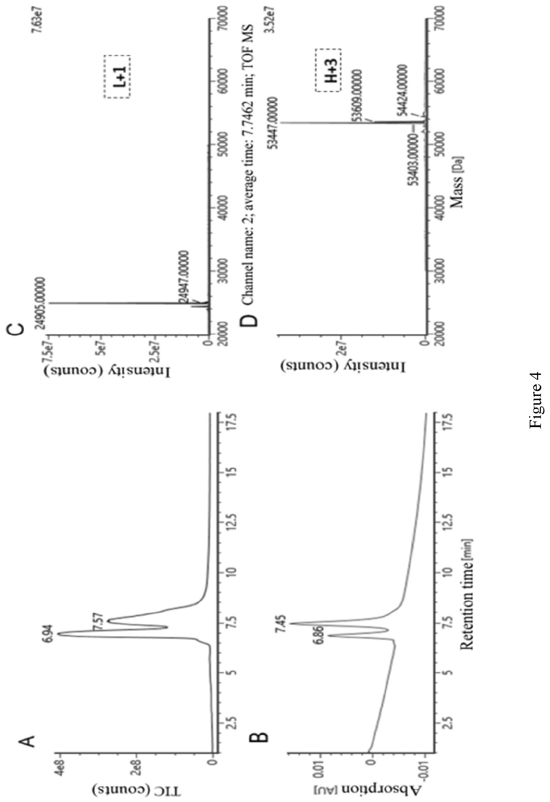

Compound 15 (0.99 mg, 0.93 mmol) was dissolved in 0.1 mL of DMA, added to the above solution system, uniformly mixed, and reacted on a rotary table at room temperature for 16 h. After the reaction was complete, small molecules were removed by using NAP-5 gel column (Cytiva), and the buffer was exchanged to a 20 mM histidine-histidine hydrochloride solution (pH = 6.2) to give the antibody-drug conjugate ADC4 (3.0mg/mL, 2 mL). The RP-HPLC-MS test results thereof were shown in FIG. 4, wherein A and B were the HPLC results, and C and D were the MS results. The average value calculated based on RP-MS was n = 7.9. The MS results showed that one linker-payload was linked to the light chain (L), and three linker-payloads were linked to the heavy chain (H).

### Example 12: Preparation of anti-IP140B antibody-drug conjugate ADC5

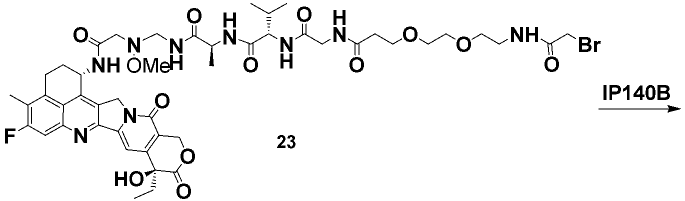

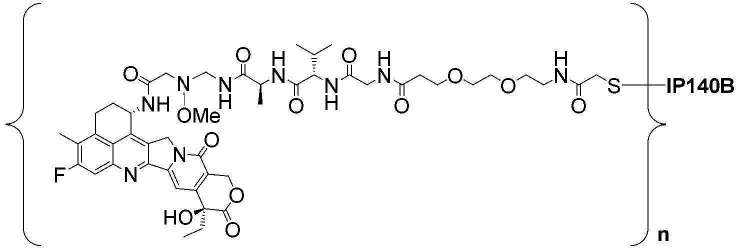

Anti-IP140B antibody (10.0 mg/mL, 10 mg, 0.066 mmol) was taken and adjusted to pH 7.2 with a 1M Na₂HPO₄ solution. A 0.1M disodium ethylenediaminetetraacetate solution (25 µL) was then added. A prepared TCEP·HCl (tris(2-carboxyethyl)phosphine hydrochloride) solution (10 mM, 0.04 mL) was added. The mixture was reacted on a rotary table at room temperature 25°C for 4 h.

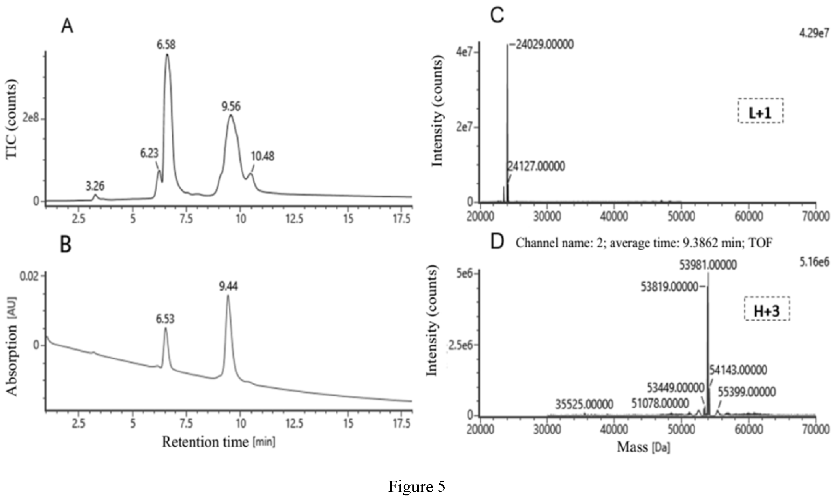

Compound 23 (0.99 mg, 0.93 mmol) was dissolved in 0.1 mL of DMA, added to the above solution system, uniformly mixed, and reacted on a rotary table at room temperature for 16 h. After the reaction was complete, small molecules were removed by using NAP-5 gel column (Cytiva), and the buffer was exchanged to a 20 mM histidine-histidine hydrochloride solution (pH = 6.2) to give the antibody-drug conjugate ADC5 (3.2 mg/mL, 2 mL). The RP-HPLC-MS test results thereof were shown in FIG. 5, wherein A and B were the HPLC results, and C and D were the MS results. The average value calculated based on RP-MS was n = 7.8. The MS results showed that one linker-payload was linked to the light chain (L), and three linker-payloads were linked to the heavy chain (H).

### Example 13: Preparation of anti-HS627 antibody-drug conjugate ADC6

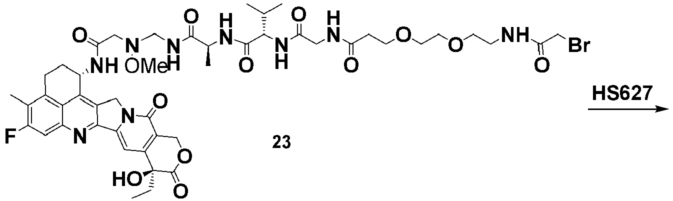

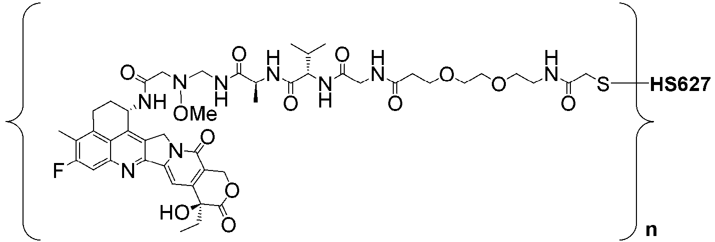

Anti-HS627 antibody (10.0 mg/mL, 10 mg, 0.066 mmol) was taken and adjusted to pH 7.2 with a 1M Na₂HPO₄ solution. A 0.1M disodium ethylenediaminetetraacetate solution (25 µL) was then added. A prepared TCEP·HCl (tris(2-carboxyethyl)phosphine hydrochloride) solution (10 mM, 0.04 mL) was added. The mixture was reacted on a rotary table at room temperature 25°C for 4 h.

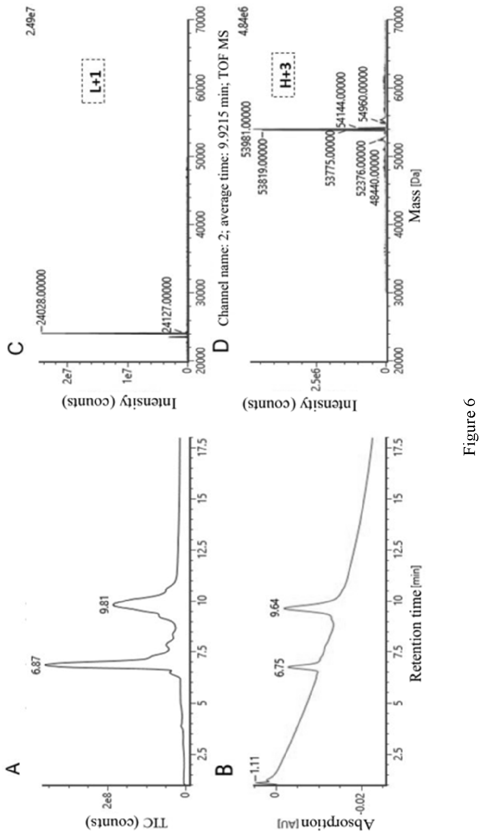

Compound 23 (0.99 mg, 0.93 mmol) was dissolved in 0.1 mL of DMA, added to the above solution system, uniformly mixed, and reacted on a rotary table at room temperature for 16 h. After the reaction was complete, small molecules were removed by using NAP-5 gel column (Cytiva), and the buffer was exchanged to a 20 mM histidine-histidine hydrochloride solution (pH = 6.2) to give the antibody-drug conjugate ADC6 (3.0 mg/mL, 2 mL). The RP-HPLC-MS test results thereof were shown in FIG. 6, wherein A and B were the HPLC results, and C and D were the MS results. The average value calculated based on RP-MS was n = 7.8. The MS showed that one linker-payload was linked to the light chain (L), and three linker-payloads were linked to the heavy chain (H).

### Example 14: Preparation of anti-Nectin-4 antibody-drug conjugate ADC7

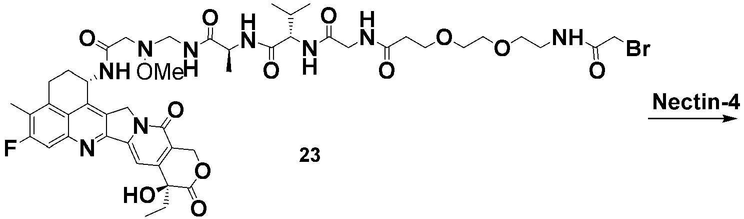

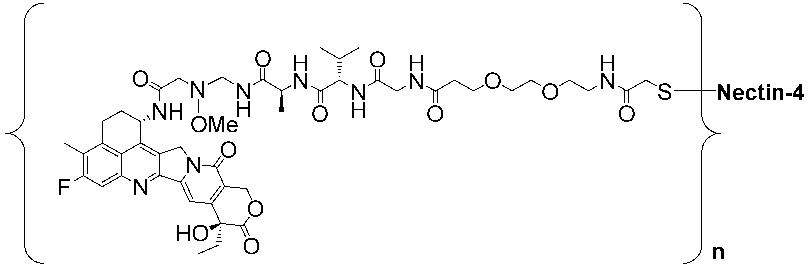

Anti-Nectin-4 antibody (10.0 mg/mL, 10 mg, 0.066 mmol) was taken and adjusted to pH 6.2 with a 1M Na₂HPO₄ solution. A 0.1M disodium ethylenediaminetetraacetate solution (25 µL) was then added. A prepared TCEP·HCl (tris(2-carboxyethyl)phosphine hydrochloride) solution (10 mM, 0.04 mL) was added. The mixture was reacted on a rotary table at room temperature 25°C for 4 h.

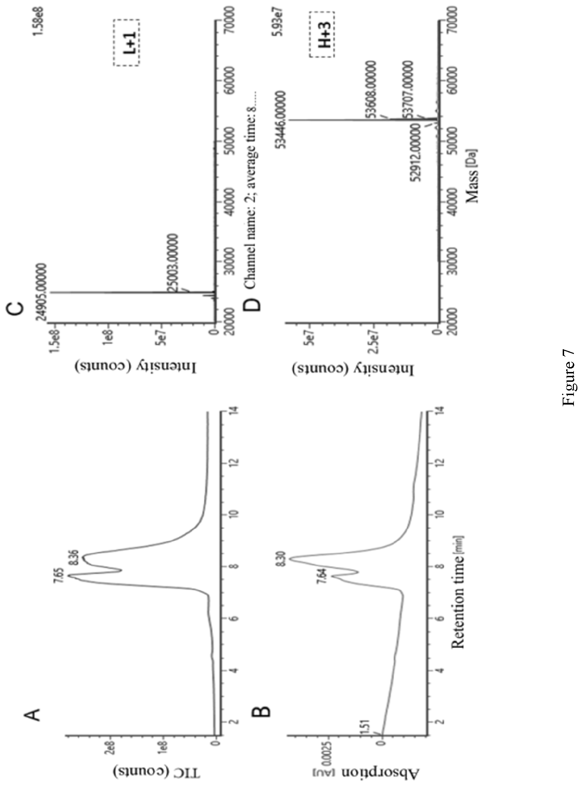

Compound 23 (0.99 mg, 0.93 mmol) was dissolved in 0.1 mL of DMA, added to the above solution system, uniformly mixed, and reacted on a rotary table at room temperature for 16 h. After the reaction was complete, small molecules were removed by using NAP-5 gel column (Cytiva), and the buffer was exchanged to a 20 mM histidine-histidine hydrochloride solution (pH = 6.2) to give the antibody-drug conjugate ADC7 (3.0 mg/mL, 2 mL). The RP-HPLC-MS test results thereof were shown in FIG. 7, wherein A and B were the HPLC results, and C and D were the MS results. The average value calculated based on RP-MS was n = 7.9. The MS results showed that one linker-payload was linked to the light chain (L), and three linker-payloads were linked to the heavy chain (H).

### Test Example 1: Inhibition of tumor growth activity in vivo by ADCs

Method for testing tumor inhibitory activity of ADC in nude mouse xenograft model (cell-derived xenograft, CDX): Pancreatic cancer cell BXPC-3, lung adenocarcinoma cell NCI-H1975, triple-negative breast cancer cell MDA-MB-231, and esophageal squamous carcinoma cell KYSE-150 were subjected to monolayer culturing in vitro. When cell saturation reached 80%-90%, the cells were digested with trypsin-EDTA and centrifuged. The supernatant was discarded. The cells were re-suspended in PBS. The cell suspension was adjusted to an appropriate concentration. BXPC-3, NCI-H1975, MDA-MB-231, and KYSE-150 cells (2-10 × 10⁶ cells/0.1 mL) were separately subcutaneously inoculated into BALB/c nude mice. The animals and the growth of transplanted tumors were observed regularly. When the tumor volume reached approximately 100-200 mm³, the mice were randomly divided into groups based on tumor volume and body weight, with 6 animals per group. The animals were administered regularly via intravenous injection. Twice a week, the longer diameter a (mm) and shorter diameter b (mm) of the tumor were measured with a vernier caliper and the body weight of the mice was measured. The tumor volume (V) was calculated according to the following formula: V = 1/2×a×b² (mm³), in which a and b represented the length and width of the tumor, respectively. A growth curve was plotted. Finally, the tumor was excised and weighed. Statistical analysis was performed based on the tumor volume and body weight data of tumor-bearing mice at the end of the experiment by using GraphPad Prism software to obtain tumor inhibition results.

The primary evaluation indicator was:
tumor growth inhibition (TGI):
TGI (%) = [1-(avTᵢ₋₀/avCᵢ₋₀)]×100%, in which avTᵢ₋₀ was the average tumor volume of the administration group on a specified day minus the average tumor volume of the administration group on the day of starting administration, and avCi-0 was the average tumor volume of the vehicle control group on the specified day minus the average tumor volume of the vehicle control group on the day of starting administration.

### 1. BXPC-3 CDX model

| | Administered drug | Administered dose | Frequency of administration | Tumor inhibition on D33 |
|---|---|---|---|---|
| Control group | Normal saline | 5 µL/g | BIW x 3 weeks | - |
| | Gemcitabine | 90 mg/kg | BIW x 3 weeks | 41.52% |
| | IgG1-Compound 15 | 5 mg/kg | BIW x 3 weeks | 24.67% |
| | IP140B | 5 mg/kg | BIW x 3 weeks | 30.66% |
| | Compound 10 | 0.12 mg/kg | BIW x 3 weeks | 16.75% |
| | IP140B+Compound 10 | 5mg/kg+0.12mg/kg | BIW x 3 weeks | 17.89% |
| Experimental group | ADC1 | 0.8 mg/kg | BIW x 3 weeks | 66.37% |
| | | 2 mg/kg | BIW x 3 weeks | 93.04% |
| | | 5 mg/kg | BIW x 3 weeks | 96.87% |

BIW was administration twice weekly. Administration was performed on Days 0, 3, 7, 10, 14, and 17. Observation was continued until Day 33.

IgG1 was a monoclonal antibody tool sequence without target selectivity, which has no affinity for human cells.

Compound 10 was a toxin (anti-tumor compound) moiety of Compound 15, and 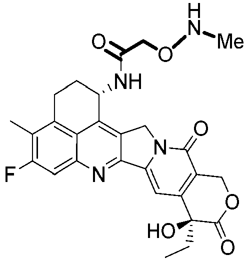 the structural formula thereof was 10.

Experimental results: The three doses 0.8 mg/kg, 2 mg/kg, and 5 mg/kg of ADC1 all demonstrated stronger tumor growth inhibitory activity and dose-dependent inhibitory activity than the positive reference Gemcitabine, the antibody alone, the toxin alone, a simple mixture of the antibody and toxin, and the ADC lacking targeting ability.

### 2. NCI-H1975 model

| | Administered drug | Administered dose | Frequency of administration | Tumor inhibition on D21 |
|---|---|---|---|---|
| Control | Normal saline | 5 µL/g | BIW x 2 weeks | - |
| | Paclitaxel | 10 mg/kg | BIW x 2 weeks | 94.5% |
| | IgG1-Compound 15 | 6 mg/kg | BIW x 2 weeks | 94.3% |
| | IP140B+Compound 10 | 6mg/kg+0.17mg/kg | BIW x 2 weeks | 11.2% |
| Experimental group | ADC1 | 1 mg/kg | BIW x 2 weeks | 96.9% |
| | | 3 mg/kg | BIW x 2 weeks | 99.4% |
| | | 6 mg/kg | BIW x 2 weeks | 99.7% |

BIW was administration twice weekly. Administration was performed on Days 0, 4, 7, and 11. Observation was continued until Day 21.

Experimental results: The three doses of 1 mg/kg, 3 mg/kg, and 6 mg/kg in the experimental group all demonstrated stronger tumor growth inhibitory activity than the simple mixture of the antibody and toxin and the ADC lacking targeting ability, and the tumor inhibitory effect of the 1 mg/kg ADC1 group was even superior to that of 10 mg/kg paclitaxel. The two dosage groups of 3 mg/kg and 6 mg/kg almost completely inhibited tumor growth, and 50% of tumor-bearing mice in the 6 mg/kg dose group had complete tumor regression.

### 3. MDA-MB-231 CDX model

| | Administered drug | Administered dose | Frequency of administration | Tumor inhibition on D25 |
|---|---|---|---|---|
| Control | Normal saline | 5 µL/g | BIW×3 doses | - |
| | Paclitaxel | 10 mg/kg | BIW×3 doses | 79.96% |
| | IgG1-Compound 15 | 6 mg/kg | BIW×3 doses | 95.93% |
| | IP140B+Compound 10 | 5mg/kg+0.12mg/kg | BIW×3 doses | 31.13% |
| | IP140B-Dxd | 3 mg/kg | BIW×3 doses | 93.05% |
| Experimental group | ADC1 | 1 mg/kg | BIW×3 doses | 72.95% |
| | | 3 mg/kg | BIW×3 doses | 93.47% |
| | | 6 mg/kg | BIW×3 doses | 95.78% |
| | ADC2 | 6 mg/kg | BIW×3 doses | 96.50% |
| | | 12 mg/kg | BIW×3 doses | 96.83% |
| | ADC5 | 3 mg/kg | BIW×3 doses | 94.17% |

BIW was administration twice weekly. Administration was performed on Days 0, 3, and 7. Observation was continued until Day 25.

The structural formula of IP140B-Dxd was (wherein n = 7.89):

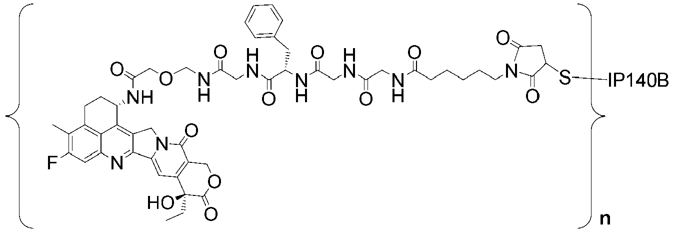

Experimental results: The two doses of 3 mg/kg and 6 mg/kg of ADC1, 3 mg/kg of ADC5, and the two doses of 6 mg/kg and 12 mg/kg of ADC2 all demonstrated stronger tumor growth inhibitory activity than the positive reference paclitaxel, the simple mixture of the antibody and toxin, and the ADC lacking targeting ability.

### 4. KYSE-150 CDX model

| | Administered drug | Administered dose | Frequency of administration | Tumor inhibition on D21 |
|---|---|---|---|---|
| Control | Normal saline | 10 µL/g | BIW x 2 weeks | - |
| | Paclitaxel | 10 mg/kg | BIW x 2 weeks | 78.00% |
| | IgG1-Compound 15 | 6 mg/kg | BIW x 2 weeks | 83.87% |
| Experimental group | ADC1 | 1 mg/kg | BIW x 2 weeks | 98.98% |
| | | 3 mg/kg | BIW x 2 weeks | 107.52% |
| | | 6 mg/kg | BIW x 2 weeks | 108.18% |
| | ADC2 | 6 mg/kg | BIW x 2 weeks | 96.92% |
| | | 12 mg/kg | BIW x 2 weeks | 98.37% |

BIW was administration twice weekly. Administration was performed on Days 0, 3, 7, and 10. Observation was continued until Day 21.

Experimental results: The three doses of 1 mg/kg, 3 mg/kg, and 6 mg/kg of ADC1 and the two doses of 6 mg/kg and 12 mg/kg of ADC2 all demonstrated stronger tumor growth inhibitory activity than the positive reference paclitaxel and the ADC lacking targeting ability.

### Test Example 2: Toxicological study of ADC in cynomolgus monkeys

### 1. Single administration of ADC to evaluate dose tolerance, pharmacokinetics, and toxicity

Following the single intravenous infusion of the ADC in cynomolgus monkeys, the pharmacokinetic properties thereof in the monkeys were observed, while the toxicity profile in the animals were observed.

### Experimental method:

In this experiment, two groups were set up, i.e., 22/35 mg/kg and 60 mg/kg groups, respectively, with two monkeys in each group, one male and one female (in the low-dose group, the 1st and 2nd administered doses were 22 mg/kg, and the 3rd and 4th administered doses were 35 mg/kg). The cynomolgus monkeys in each group were both intravenously injected with the ADC formulation having the corresponding concentration, based on an administration volume of 5 mL/kg. The administration was performed once every two weeks for two consecutive administrations, followed by washout and recovery for 22 days. Then, a 3rd administration was performed, and after two weeks, a 4th administration was administered. For the 22/35 mg/kg group, the day of the first administration was defined as Experimental Day 1. For the 60 mg/kg group, the day of the first administration was defined as Experimental Day 5. The cynomolgus monkeys were observed daily for general condition during the administration period, and the body weight and food intake were measured once weekly. The cynomolgus monkeys in each group three days after the first administration and the cynomolgus monkeys in the 22/35 mg/kg group on Experimental Days 19, 42, 56, and 66 were subjected to venous blood sampling for hematology and blood biochemistry examinations. On Experimental Day 66, the cynomolgus monkeys in the 22/35 mg/kg group were anesthetized and euthanized for gross necropsy observation, organ weight measurement, and bone marrow smears. Histopathological examination was performed on the heart, liver, spleen, lungs, kidneys, ovaries, cervix, epididymis, inguinal lymph nodes, and site of administration (including blood vessels) of the cynomolgus monkeys in each group.

Pharmacokinetics: Following single intravenous infusion of different dose of the ADC in cynomolgus monkeys, blood samples were collected serially at multiple timepoints. Blood samples were collected from the cynomolgus monkeys in the 22/35 mg/kg group via the upper limb vein prior to the 1st and 3rd administrations; at 0.0167 hours after the end of the 2nd administration; and at 0.167, 4, 24, 48, 72, 96, 168, and 336 hours after the end of the 1st, 3rd, and 4th administrations. The serum concentrations of ADC-TAB were detected by an ELISA method, and the plasma concentrations of the small-molecule toxin were detected by an LC-MS/MS method. Toxicokinetic parameters such as AUClast were calculated.

Toxicity study: Following the single intravenous infusion of different doses of the ADC in cynomolgus monkeys, the tolerance in the animals and the drug-related toxicity profile were observed in various aspects such as clinical observations, body weight and food intake, hematology, blood biochemistry, urine, and gross anatomy.

### Experimental results:

During the experiment, the cynomolgus monkeys were in good general condition, exhibited normal spontaneous activity, and showed no significant toxic reactions. During the administration period, no significant abnormal changes in food intake were found in the male and female monkeys in the 22/35 mg/kg group, no abnormalities in body weight were found in the cynomolgus monkeys after two consecutive administrations at 22 mg/kg, and when the dose was increased to 35 mg/kg, no significant changes in body weight were found in the cynomolgus monkeys after two consecutive administrations. A slight decrease in food intake and body weight was found in the cynomolgus monkeys in the 60 mg/kg group 3-4 days after the first administration.

At all test timepoints during the experiment, no significant abnormal changes in biochemical indicators were found in the blood of the female and male cynomolgus monkeys in each group.

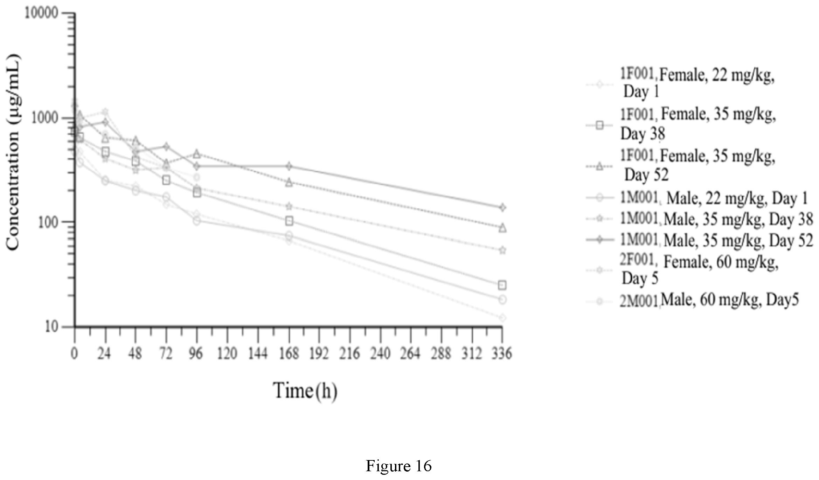

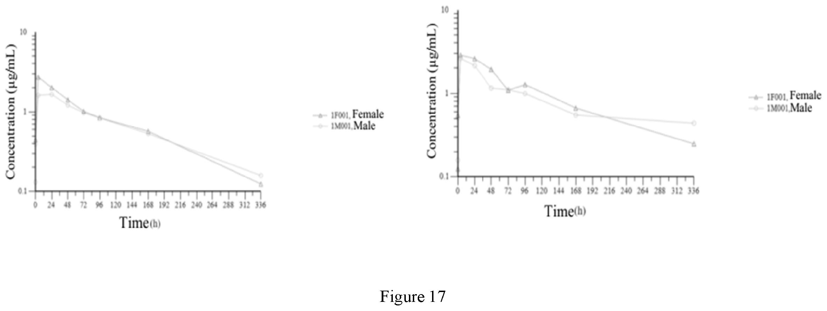

Pharmacokinetics: Following the single intravenous infusion of ADC in cynomolgus monkeys, the ADC was stable in the blood of the cynomolgus monkeys (see FIG. 16), and the free toxin concentration was low (see FIG. 17), indicating that the ADC underwent slow release in cynomolgus monkeys and the conjugation was stable.

Toxicity profile: Following the single intravenous infusion of ADC in cynomolgus monkeys, the tolerance in the animals was good, and no severe or intolerable drug-related toxicity was exhibited, indicating that the safety of the ADC was controllable and could support its further clinical research.

### 2. Repeated administration to investigate potential target organs of toxicity and pharmacokinetic parameters

A toxicity experiment in cynomolgus monkeys involving intravenous injection once every three weeks for six consecutive weeks (three administrations in total) was conducted to observe the nature, extent, dose-effect and time-response relationships, and reversibility of toxic reactions that may be induced by the test article, identify the target organs or target tissues of toxicity, simultaneously study the toxicokinetic characteristics thereof, and concurrently investigate the effects on the cardiovascular system, central nervous system, respiratory function, and site of administration, providing a reference for the safety of clinical use.

### Experimental method:

In this experiment, 4 groups were set up, which were respectively a control group (ADC5 formulation buffer) and ADC5 @ 9, 18, and 36 mg/kg groups, with 10 cynomolgus monkeys in each group, half male and half female. The cynomolgus monkeys in each group were intravenously injected with the control (ADC5 formulation buffer) or ADC5 having the corresponding concentration, based on a volume of 5 mL/kg. Administration was performed once every three weeks for six consecutive weeks (three administrations in total), followed by dose-free recovery observation for six weeks. The day of the first administration was Day 1 of the administration period, and the day after the last administration was Day 1 of the recovery period.

Blood samples were collected from the cynomolgus monkeys in each group separately before the first and last administrations and immediately after the first and last administrations (0-2 minutes after the end of administration), at 4, 8, 24, 48, 72, 96, 168, 240, 336, and 504 hours after administration, and at 672 hours after the end of the last administration. The serum concentrations of ADC-TAB (total antibodies) and ADC (antibody-drug conjugate) in the monkeys at each timepoint were detected by an ELISA method. The plasma concentrations of free toxins in the cynomolgus monkeys at each timepoint were detected by an LC-MS/MS method. Toxicokinetic parameters such as AUC were calculated.

### Experimental results:

During the administration period, a decrease in food intake was observed in some female and male cynomolgus monkeys in the 18 and 36 mg/kg groups. In addition, during the administration period and the recovery period, no significant abnormal changes in food intake were found in the female and male cynomolgus monkeys in the 9 mg/kg group, and no significant abnormal changes in body weight were found in the female and male cynomolgus monkeys in each ADC5 group.

At 4-5, 24, 72, 168, 336, and 504 hours after the first administration, at 4 hours after the last administration, and at the end of the recovery period, no arrhythmia was found in the lead II electrocardiogram of the female and male cynomolgus monkeys in each ADC5 group, and lead II electrocardiogram indicators such as heart rate, P-wave time, PR interval, QRS time, QT interval, RR interval, and corrected QT interval, and indicators related to blood pressure and respiration detection, showed no significant abnormalities. No significant abnormalities were found in detection-related indicators such as various blood biochemical indicators, urine indicators, lymphocytes, cytokines, bone marrow smears, and organs.

### Pharmacokinetics:

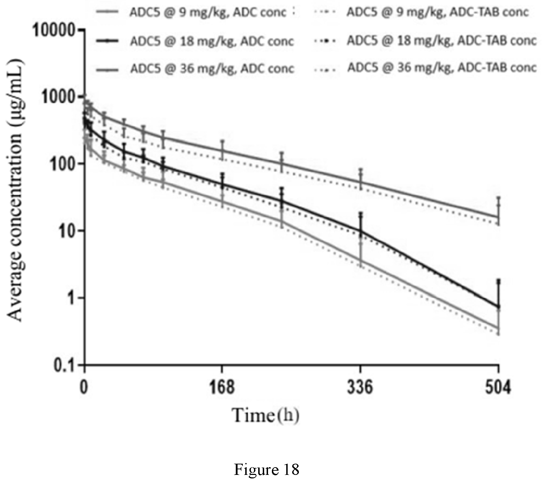

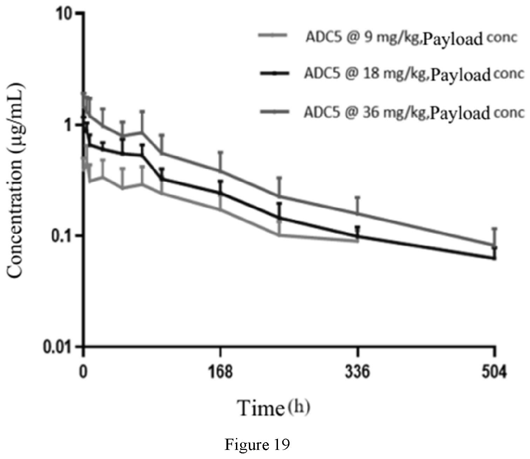

Under the conditions of this experiment, cynomolgus monkeys received intravenous injection with ADC5 at 9, 18, and 36 mg/kg once every three weeks for six consecutive weeks (three administrations in total), followed by dose-free recovery for six weeks. ADC5 was stable in the blood of the cynomolgus monkeys (see FIG. 18), and the free toxin concentration was low (see FIG. 19), indicating that the ADC underwent slow release in cynomolgus monkeys and the conjugation was stable, supporting a clinical Q3W administration regimen.

Toxicity profile: Following the repeated intravenous infusion of the ADC in cynomolgus monkeys, the tolerance in the animals was good, mild manifestations such as intermittent reduction in food could all recover, and no severe or intolerable drug-related toxicity was exhibited, indicating that the safety of the ADC was controllable and could support its further clinical research.

## Claims

1. An antibody-drug conjugate represented by formula (I): 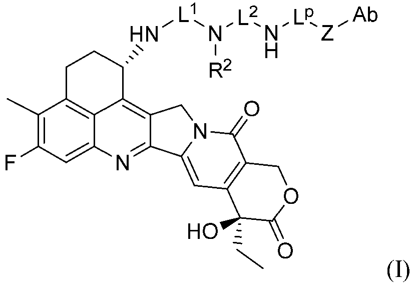 in the formula,
R² is selected from hydrogen, deuterium, C₁-C₆ alkyl, C₁-C₆ alkoxy, acyl, or sulfonyl;
L¹ is selected from -L¹¹-L¹²-L¹³-, wherein L¹¹, L¹², and L¹³ are each independently selected from absence, -C=O-, C₁-C₂ alkylene, or C₁-C₂ alkylene-O-, provided that when L¹¹ is - C=O-, R² is not hydrogen;
L² is selected from C₁-C₆ alkylene or C₁-C₆ acyl, the C₁-C₆ alkylene or C₁-C₆ acyl being optionally substituted with one or more R³;
R³ is selected from phenyl-substituted or unsubstituted C₁-C₆ alkyl or C₁-C₆ alkoxy;
L^{P} is a peptide residue composed of 1-7 amino acids;
Z is selected from -L^{z}-L^{j}-, wherein L^{z} is selected from absence, -C(=O)-C₁-C₈ alkylene, - C(=O)-(CH₂CH₂O)₂₋₆-CH₂CH₂NH-, or -C(=O)-(CH₂)₀₋₆-NR¹(CH₂)₀₋₆-, and L^{j} is an antibody-conjugatable linker;
R¹ is selected from -C₁-C₆ alkyl-carboxyl or -C₁-C₆ alkyl-amino; and
Ab is an antibody.

2. The antibody-drug conjugate according to claim 1, wherein R² is selected from hydrogen, deuterium, C₁-C₃ alkyl, C₁-C₃ alkoxy, -C(=O)C₁-C₃ alkyl, or -S(=O)₂C₁-C₃ alkyl;
preferably, R² is selected from hydrogen, deuterium, methyl, ethyl, methoxy, ethoxy, formyl, acetyl, methylsulfonyl, or ethylsulfonyl;
preferably, R² is selected from hydrogen, methyl, methoxy, formyl, or methylsulfonyl.

3. The antibody-drug conjugate according to claim 1 or 2, wherein L¹¹, L¹², and L¹³ are each independently selected from absence, -C=O-, -CH₂-, -CH₂O-, or -OCH₂-;
preferably, L¹ is selected from -C(=O)CH₂OCH₂-, -C(=O)CH₂O-, -C(=O)CH₂, or -CH₂-;
preferably, L² is selected from C₁-C₃ alkylene;
preferably, L² is selected from methylene or ethylene.

4. The antibody-drug conjugate according to any one of claims 1-3, wherein L^{p} is selected from a peptide residue composed of 1, 2, 3, or 4 amino acids selected from phenylalanine (Phe), glycine (Gly), valine (Val), alanine (Ala), or leucine (Leu);
preferably, L^{p} is selected from -Val-Cit-, -Gly-Lys-, -Gly-Leu-, -Val-Ala-, -Gly-Phe-, - GLy-Gly-Lys-, -Gly-Gly-Phe-, -Gly-Val-Ala-, -Gly-Ala-, -Gly-Gly-Val-, -Gly-Leu-Val-, -Gly-Phe-Gly-, or -Gly-Gly-Leu-;
preferably, L^{p} is selected from

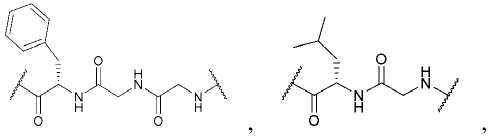

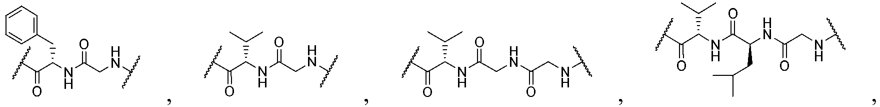

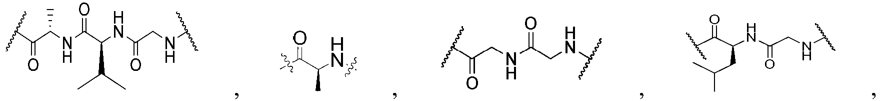

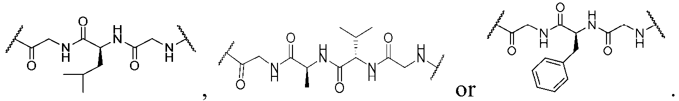

5. The antibody-drug conjugate according to any one of claims 1-4, wherein L^{j} is selected from 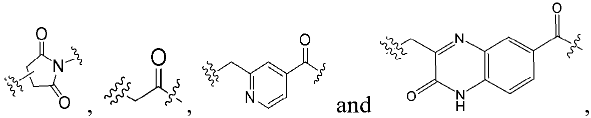 the position represented by 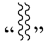 indicating where the antibody is linked, and the position represented by 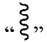 indicating where the L^{z} group is linked;
preferably, L^{z} is selected from -C(=O)-C₁-C₈ alkylene, -C(=O)-(CH₂CH₂O)₂₋₆-CH₂CH₂NH-, or -C(=O)-(CH₂)₁₋₄-NR¹(CH₂)₂-;
preferably, L^{z} is selected from -C(=O)-(CH₂CH₂O)₂₋₆-CH₂CH₂NH- or -C(=O)-(CH₂)₁₋₄-NR¹(CH₂)₂-;
preferably, R¹ is selected from -C₁-C₃ alkyl-carboxyl;
preferably, L^{z} is selected from -C(=O)-(CH₂CH₂O)₂-CH₂CH₂NH- or 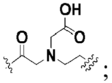
preferably, Z is selected from

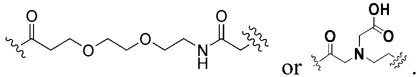

6. The antibody-drug conjugate according to any one of claims 1-5, wherein the antibody-drug conjugate is obtained by conjugating a compound of formula (II) or (III) to the antibody: 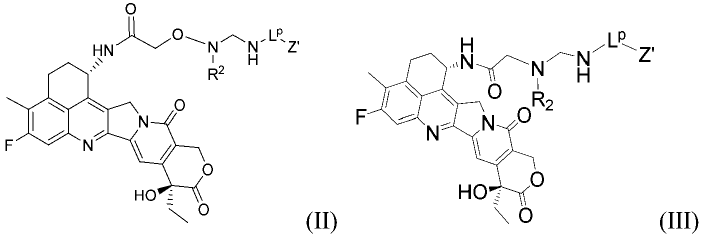
Wherein, R² and L^{P} are each the same as defined for the compound of formula (I), Z' is an antibody-conjugatable linker group corresponding to Z, preferably maleimido, bromoacetyl, or iodoacetyl;
preferably, the antibody-drug conjugate releases the following anti-tumor compounds in a physiological environment: 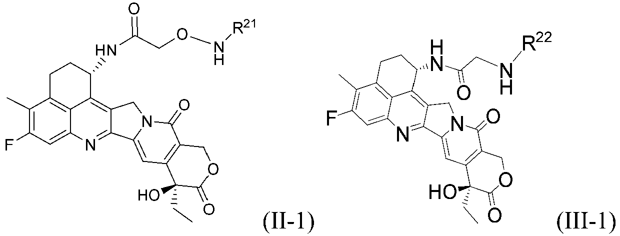
Wherein, R²¹ is selected from C₁-C₆ alkyl, preferably -C₁-C₃ alkyl, more preferably methyl; R²² is selected from C₁-C₆ alkoxy, preferably -C₁-C₃ alkoxy, more preferably methoxy;
preferably, the following anti-tumor compounds are released:

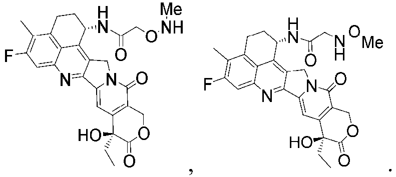

7. The antibody-drug conjugate according to any one of claims 1-6, wherein the antibody is an antibody against a tumor-associated antigen;
preferably, the tumor-associated antigen is selected from one or more antibodies of Her2, Nectin-4, Trop2, 5T4, B7H3, ROR1, or Claudin18.2;
preferably, the tumor is selected from breast cancer, lung cancer, colorectal cancer, esophageal cancer, gastric cancer, lung cancer, renal cancer, ovarian cancer, cervical cancer, bladder cancer, head and neck cancer, pancreatic cancer, or liver cancer;
preferably, the antibody comprises a heavy chain having an amino acid sequence of SEQ ID NO: 1 or any variant thereof, and a light chain having an amino acid sequence of SEQ ID NO: 2 or any variant thereof;
preferably, the antibody comprises a heavy chain having an amino acid sequence of SEQ ID NO: 3 or any variant thereof, and a light chain having an amino acid sequence of SEQ ID NO: 4 or any variant thereof;
preferably, the antibody comprises a heavy chain having an amino acid sequence of SEQ ID NO: 5 or any variant thereof, and a light chain having an amino acid sequence of SEQ ID NO: 6 or any variant thereof;
preferably, the drug-to-antibody ratio of the antibody-drug conjugate is 2-8, further preferably 3.5-4.5 or 7.5-8.

8. The antibody-drug conjugate according to any one of claims 1-7, wherein the antibody-drug conjugate is obtained by conjugating the following compound to the antibody:

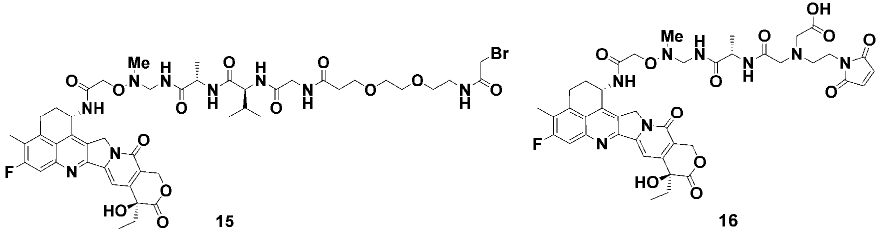

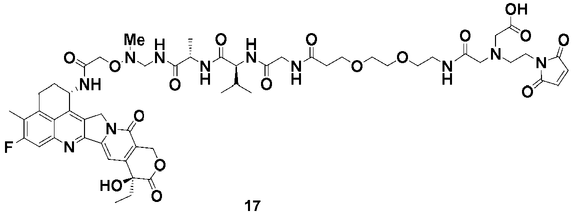

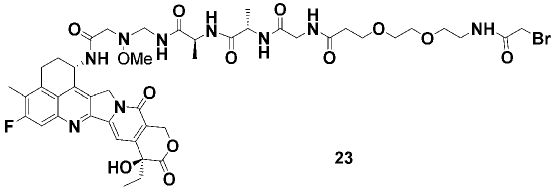

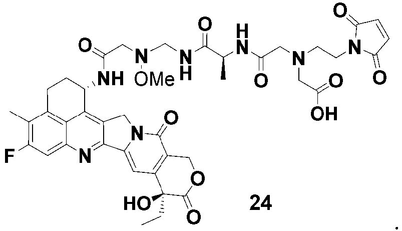

9. A pharmaceutical composition, comprising the antibody-drug conjugate according to any one of claims 1-8 and a pharmaceutically acceptable carrier.

10. Use of the antibody-drug conjugate according to any one of claims 1-8 or the pharmaceutical composition according to claim 9 in the manufacture of an anti-tumor drug, preferably, the tumor is selected from breast cancer, lung cancer, colorectal cancer, esophageal cancer, gastric cancer, lung cancer, renal cancer, ovarian cancer, cervical cancer, bladder cancer, head and neck cancer, pancreatic cancer, or liver cancer.

11. A method for treating a tumor disease, comprising a step of administering to a patient in need thereof a therapeutically effective amount of the antibody-drug conjugate according to any one of claims 1-8 or the pharmaceutical composition according to claim 9,
preferably, the tumor is a solid tumor;
preferably, the tumor is selected from breast cancer, lung cancer, colorectal cancer, esophageal cancer, gastric cancer, lung cancer, renal cancer, ovarian cancer, cervical cancer, bladder cancer, head and neck cancer, pancreatic cancer, or liver cancer.
